# EUROPEAN PATENT APPLICATION

(11) **EP 3 705 131 A1**
(43) Date of publication of application: **09.09.2020**
(21) Application number: 19160469.3
(22) Date of filing: 04.03.2019
(51) Int. Cl.: A61K 38/16, C07K 14/31

(54) **LANTIBIOTICS AND LANTIBIOTIC-PRODUCING BACTERIA**

(71) Applicant: University College Cork-National University of Ireland, Cork, Cork (IE); Agriculture and Food Development Authority (TEAGASC), Co. Carlow (IE)
(72) Inventor: ROSS, Reynolds Paul, Co Cork (IE); STANTON, Catherine, Co Cork (IE); HILL, Colin, Cork (IE); RYAN, Anothony, Cork (IE); O'SULLIVAN, Julie, Cork (IE); WATKINS, Claire, Cork (IE)
(74) Representative: Murgitroyd & Company

(57) **Abstract**

An isolated lantibiotic is provided comprising amino acid sequence SEQ. ID No. 2 and at least one of amino acid sequences SEQ. ID No. 4 and SEQ. ID. No 6. Also provided is use of the lantibiotic in the treatment or prevention of infection by Group B Streptococcus or Corynebacterium xerosis. The invention further relates to isolated S. hominis strains and use of same in producing the lantibiotic. A vector is provided comprising nucleotides sequences for producing the lantibiotic.

## Description

### Field of the Invention

The present invention relates to lantibiotics and to lantibiotic-producing bacteria, including methods of making the lantibiotics and methods of using the lantibiotics in the treatment and prevention of bacterial infection, in particular infection with Group B Streptococcus (GBS).

### Background of the Invention

Given the public health threat posed by antibiotic resistance, alternative prophylactic options are currently being explored. Ribosomally synthesised antimicrobial peptides of bacterial origin, also known as bacteriocins, with narrow to broad spectrum activity are known to be effective in the prevention and treatment of pathogenic microorganisms, alongside other alternatives such as phage therapy, vaccines, probiotics and endo- or exolysins. Bacteriocins can be classified as either class I, post-translational modified peptides, or class II, largely unmodified peptides. Ribosomally synthesised, Post translationally modified Peptides (RiPPs) have also been adopted as a suitable nomenclature for all modified (class I) peptides. Lantibiotics (lanthionine-containing antibiotics) are a subgroup of RiPPs which involve the formation of lanthionine (Lan) and methyllanthionine (MeLan) bridges. The dehydration of serine (Ser) and threonine (Thr) residues to form 2,3-didehydroalanine (Dha) and 2,3-didehydrobutyrine (Dhb) residues, respectively, is followed by reaction with a cysteine (Cys) residue to form either a Lan or a MeLan bridge. The formation of Lan/MeLan bridges is catalysed by a multifunctional LanM modification enzyme in type II (class IB) lantibiotics such as lacticin 3147, licheniciden, formicin and mersacidin. However, in type I (class IA) lantibiotics, such as nisin and epidermin, this modification is performed by separate LanB and LanC enzymes.

Alongside the previously mentioned peptide modification enzymes (LanM, LanB and LanC), there are a number of other genes essential for lantibiotic synthesis. These genes are involved in either the biosynthesis of or immunity to lantibiotics. Following post translational modification of the structural peptides, ABC (ATP binding cassette) transporters (LanT) are present to export the lantibiotic from the cell. This exporting process can also provide immunity (LanFEG) to the cell by pumping the lantibiotic out and away from the cell wall, preventing self-damage. During this stage of lantibiotic production, a proteolytic process is carried out either during or after transport by a protease (lanP) enzyme, which cleaves the leader peptide from the active core-peptide.

Within the lantibiotic group, type IB, is a subgroup of two-component lantibiotics. This interesting cluster requires a gene cassette with two structural peptides, described as α and β peptides, which work synergistically to produce antimicrobial activity. The mode of action in two-component lantibiotic systems is of particular interest where two LanM modification enzymes are required to catalyse each prepeptide individually in the gene cassette. For example, in lacticin 3147, antimicrobial activity occurs when the α peptide binds to lipid II in the peptidoglycan cell wall and inhibition of cell wall synthesis occurs only when the β peptide is present, forming small pores in the cell membrane. Previously identified two-component lantibiotics have been isolated from a range of different microorganisms including Staphylococcus aureus (staphylococcin C55) (Navaratna et al., 1998), Lactococcus lactis (lacticin 3147) (Ryan et al., 1996), Lactobacillus plantarum (plantaricin W)(Holo et al., 2001), Bacillus licheniformis (lichenicidin) (Begley et al., 2009), Bacillus halodurans (haloduracin) (McClerren et al., 2006), Enterococcus faecalis (enterocin W) (Sawa et al., 2012) and Bacillus paralicheniformis (formicin) (Collins et al., 2016). In addition to eliminating numerous pathogens, these lanthipeptides are considered a probiotic trait due to their ability to modulate the composition of the microbiota and influence host immune systems.

Group B Streptococcus (GBS), also known as Streptococcus agalactiae, is a gram-positive, coccus shaped, catalase negative commensal organism found to be resident or transient in the gastrointestinal and genitourinary tracts. It is estimated that 15-30% of woman are asymptomatic carriers of GBS, of which half will transmit GBS to their infants, leading to ∼ 2-3% of infants being born with the invasive organism. As a leading cause of neonatal infection, and indeed neonatal mortality, GBS maternal transmission is the primary route of the disease. Current recommendations to reduce the rate of infection include late-gestational screening for GBS in pregnant women and intrapartum antibiotic prophylaxis (IAP) for GBS-positive mothers. Intravenous administration of ampicillin, penicillin, clindamycin or vancomycin at the onset of labour have been found to be effective in the prevention of GBS transmission from mother to infant. However, IAP during labour can have a significant impact on the infant microbiome. Moreover, a Cochrane review carried out to assess the impact of maternal IAP on neonatal GBS infections concluded that there is lack of evidence to recommend IAP to reduce neonatal early-onset group B streptococcal disease. There is a need therefore for a new treatment for the prophylactic prevention of GBS colonisation in both mother and infant.

Corynebacterium xerosis, a Gram positive, non-lipophilic skin commensal, has been found to colonise the nasopharynx, conjunctiva and the vagina. C. xerosis has been associated with cases of pneumonia, sepsis and conjunctivitis. However, it is considered a putative pathogen due to its non-toxic and non-motile phenotype. Typically, C. xerosis has been found in clinical specimens of immunocompromised individuals and further study is required to determine its pathology.

### Summary of the Invention

The present inventors have identified a two-component lantibiotic, named Homicin, that is produced by vaginal isolate Staphylococcus hominis APC 3675 and skin isolates Staphylococcus hominis APC 2924 and APC 2925.

Accordingly, according to a first aspect of the present invention, there is provided an isolated lantibiotic comprising:
- amino acid sequence SEQ. ID No. 2 or a variant amino acid sequence having at least 90% amino acid sequence identity thereto, and
- at least one of:
   - amino acid sequence SEQ. ID No. 4 or a variant amino acid sequence having at least 90% amino acid sequence identity thereto, and
   - amino acid sequence SEQ. ID. No 6 or a variant amino acid sequence having at least 90% amino acid sequence identity thereto.

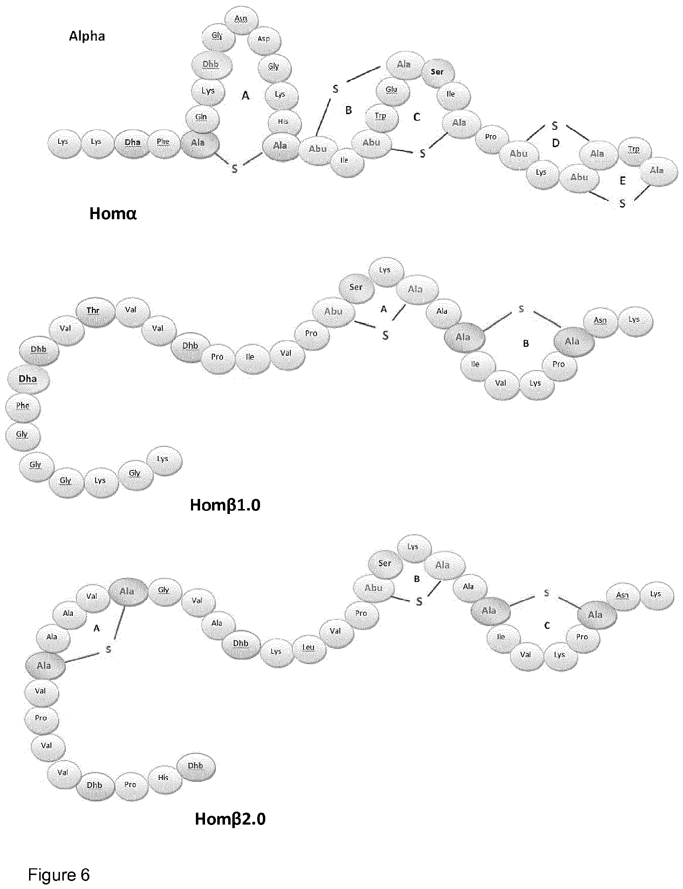

SEQ ID No. 2, 4 and 6 relate to the active core amino acid sequences of Homicin α-peptide and β-peptides (homβ1 and homβ2) respectively. The lantibiotic is a two-component lantibiotic - the α and β peptide work synergistically to produce antimicrobial activity. The structural prediction of the active two-component lantibiotic is shown in Figure 6. The two-component lantibiotic may comprise amino acid sequence SEQ. ID No. 2 and at least one of amino acid sequence SEQ. ID No. 4 and amino acid sequence SEQ. ID. No 6. The two-component lantibiotic may comprise amino acid sequence SEQ. ID. No. 2, amino acid sequence SEQ. ID. No. 4 and amino acid sequence SEQ. ID. No. 6. Alternatively, the two-component lantibiotic may comprise amino acid sequence SEQ. ID. No. 2 and amino acid sequence SEQ. ID. No. 4 or may comprise amino acid sequence SEQ. ID. No. 2 and amino acid sequence SEQ. ID. No. 6. Typically, the ratio of SEQ ID. No. 2 : SEQ ID No. 4 and/or 6 is 1:1.

The two-component lantibiotic is a Staphylococcus hominis lantibiotic. The two-component lantibiotic, or one or more of the amino acid sequences thereof is, obtainable by culturing S. hominis APC 3675 NCIMB 43358, S. hominis APC 2924 NCIMB 43356 or S. hominis APC 2925 NCIMB 43357. The two-component lantibiotic, or one or more of the amino acid sequences thereof, may be isolated from S. hominis APC 3675 NCIMB 43358, S. hominis APC 2924 NCIMB 43356 or S. hominis APC 2925 NCIMB 43357.

The invention further extends to a pharmaceutical composition comprising the two-component lantibiotic of the invention and a pharmaceutically acceptable carrier.

According to a further aspect of the invention, there is provided the two-component lantibiotic of the invention for use as a medicament.

In particular, there is provided the two-component lantibiotic of the invention for use in inhibiting or preventing the growth of a bacterium. The invention provides a method for inhibiting or preventing the growth of a bacterium comprising contacting a bacterium with an effective amount of the two-component lantibiotic of the invention. The invention further provides for use of the two-component lantibiotic of the invention in the preparation of a medicament for inhibiting or preventing the growth of a bacterium. Inhibiting or preventing the growth of a bacterium includes inhibiting or preventing colonisation of bacteria, for example, on human skin or vagina. The bacteria may be pathogenic gram positive bacteria (e.g. staphylococci or streptococci).

The two-component lantibiotic of the invention is disclosed for use as an anti-microbial agent, in particular, for preventing or treating a bacterial infection. The invention provides a method for preventing or treating a bacterial infection comprising administering to a subject in need of treatment an effective amount of the two-component lantibiotic of the invention. The invention further provides for use of the two-component lantibiotic of the invention in the preparation of a medicament for use as an anti-microbial agent, in particular, for preventing or treating a bacterial infection.

The invention further extends to isolated amino acid sequences of the two-component lantibiotic of the invention.

Accordingly, according to a further aspect of the invention there is provided an isolated amino acid sequence comprising, consisting essentially of or consisting of amino acid sequences of SEQ. ID. No: 2, SEQ ID NO: 4 or 6, or variant amino acid sequence having at least 90% amino acid sequence identity thereto.

The variant amino acid sequence may have at least 91%, 92/%, 93%, 94%, 95%, 96%, 97%, 98% or 99% sequence identity with amino acid SEQ ID No. 2, 4 or 6. The variant amino acid sequence may have 95%, 98% or 99% sequence identity with amino acid SEQ ID No. 2, 4 or 6.

The variant amino acid sequence may differ from the amino acid sequence on which it is based due to the presence of one or more conservative amino acid substitutions. Typically, the variant amino acid sequence differs from the amino acid sequence on which it is based due to the presence of less than 5, 4, 3, 2 or 1 conservative amino acid substitutions. The only differences between the variant amino acid sequence and the amino acid sequence on which it is based may be conservative amino acid substitutions, that is, no non-conservative amino acid residue alterations are present.

In certain embodiments, the variant amino acid sequence may not include any amino acid substitutions at positions identified as containing amino acid residues involved in forming a lanthionine bridge or amino acids which are dehydrated, i.e. serine (Ser) and threonine (Thr) residues which are dehydrated to form 2,3-didehydroalanine (Dha) and 2,3-didehydrobutyrine (Dhb) residues, respectively.

In certain embodiments, the variant amino acid sequence may include one or more, preferably less than 3 and more preferably less than 2, truncations, substitutions, deletions or insertions.

Typically, the two-component lantibiotic comprising one or more variant amino acid sequences as above retains anti-microbial activity. Typically, the two-component lantibiotic has the same or improved anti-microbial activity when compared to the two-component lantibiotic comprising amino acid sequence SEQ. ID No. 2 and at least one of amino acid sequence SEQ. ID No. 4 and amino acid sequence SEQ. ID. No 6, i.e. the changes to the amino acid sequences do not result in any loss of anti-microbial activity.

The invention further extends to isolated nucleic acid molecules encoding for the two-component lantibiotic of the invention. The nucleic acid molecules may encode the amino acid sequences which make up the two-component lantibiotic as described above.

Accordingly, according to a further aspect of the invention there is provided an isolated nucleotide sequence encoding a polypeptide comprising amino acid sequence SEQ. ID No. 2, 4 or 6.

The isolated nucleotide sequence of the invention may comprise, include, consist essentially of or consist of SEQ ID No.15, 17 or 19 or a variant nucleotide sequence which has at least 90% sequence identity to
(a) SEQ ID No.15, 17 or 19, or
(b) a nucleotide sequence that is capable of hybridising to SEQ ID No.15, 17 or 19 under stringent conditions.

Typically, said isolated nucleotide sequence encodes an amino acid sequence which may be combined with one or more further amino acid sequences to form a two-component lantibiotic having anti-microbial activity.

The variant nucleotide sequence may have at least 91%, 92/%, 93%, 94%, 95%, 96%, 97%, 98% or 99% sequence identity with SEQ ID No.15, 17 or 19 or a nucleotide sequence that is capable of hybridising to SEQ ID No.15, 17 or 19 under stringent conditions. The variant nucleotide sequence may have at least 95%, 98% or 99% sequence identity with SEQ ID No.15, 17 or 19 or a nucleotide sequence that is capable of hybridising to SEQ ID No.15, 17 or 19 under stringent conditions. The variant nucleotide sequence may have at least 100% sequence identity with a nucleotide sequence that is capable of hybridising to SEQ ID No.15, 17 or 19 under stringent conditions.

In certain embodiments, the isolated nucleotide sequence is obtained from S. hominis APC 3675 NCIMB 43358, S. hominis APC 2924 NCIMB 43356 or S. hominis APC 2925 NCIMB 43357. In certain embodiments, the isolated nucleotide sequence is obtainable by culturing S. hominis APC 3675 NCIMB 43358, S. hominis APC 2924 NCIMB 43356 or S. hominis APC 2925 NCIMB 43357.

Also provided is an isolated plasmid comprising a nucleotide sequence encoding a polypeptide comprising amino acid sequences SEQ. ID No. 2, 4 and 6, optionally wherein the nucleotide sequence further encodes one or more of SEQ ID No. 1, 3, 5, 7, 8, 9, 10, 11, 12 and 13. In certain embodiments, the nucleotide sequence encodes a polypeptide comprising amino acid sequences SEQ. ID No. 2, 4, 6, 1, 3, 5, 7, 8, 9, 10, 11, 12 and 13. In certain embodiments, the plasmid comprises SEQ ID No. 15, 17 and 19, optionally wherein the plasmid further comprises one or more of SEQ ID No. 14, 16, 18, 20, 21, 22, 23, 24, 25 and 26. In certain embodiments, the plasmid comprises SEQ ID No. 34, 35 and 36. In certain embodiments, the plasmid comprises SEQ ID No. 20 and 21. In certain embodiments, the plasmid comprises SEQ ID No. 22. In certain embodiments, the plasmid comprises SED ID No. 23, 24, 25 and 26. In certain embodiments, the plasmid comprises SEQ ID No. 34, 35, 36, 20, 21, 22, 23, 24, 25 and 26. The invention further extends to all strains comprising the plasmid of the invention.

The invention further extends to an isolated polypeptide encoded by an isolated nucleotide sequence of the invention. Preferably there is provided a polypeptide sequence encoded by a nucleotide sequence comprising, consisting essentially of or consisting of SEQ ID No.15, 17 or 19 or a variant nucleotide sequence as described above. Suitably a polypeptide of the invention will have anti-microbial activity when combined to form the two-component lantibiotic of the invention.

According to a further aspect of the present invention there is provided an isolated S. hominis strain selected from the group consisting of S. hominis APC 3675 deposited under NCIMB 43358, S. hominis APC 2924 deposited under NCIMB 43356 and S. hominis APC 2925 deposited under NCIMB 43357. The genome sequence for S. hominis APC 3675 is provided as SED ID No. 39. The genome sequence for S. hominis APC 2924 is provided as SED ID No. 37. The genome sequence for S. hominis APC 2925 is provided as SED ID No. 38.

Also provided is an isolated strain which produces the lantibiotic of the invention.

The invention extends to the isolated strain of the invention for use as a medicament. In particular, the invention extends to the isolated strain of the invention for use in inhibiting or preventing the growth of a bacterium. The invention provides a method for inhibiting or preventing the growth of a bacterium comprising contacting a bacterium with an effective amount of the strain of the invention. The invention further provides for use of the strain of the invention in the preparation of a medicament for inhibiting or preventing the growth of a bacterium. Inhibiting or preventing the growth of a bacterium includes inhibiting or preventing colonisation of bacteria, for example, on human skin or vagina. The bacteria may be pathogenic gram positive bacteria (e.g. staphylococci or streptococci).

The invention further extends to the isolated strain of the invention for use in preventing or treating a bacterial infection. The invention provides a method for preventing or treating a bacterial infection comprising administering to a subject in need of treatment an effective amount of the strain of the invention. The invention further provides for use of the strain of the invention in the preparation of a medicament for preventing or treating a bacterial infection.

The invention further extends to the isolated strain of the invention for use as a probiotic food and to a probiotic food comprising at least one of the isolated strains of the invention.

The isolated strain of the invention may be provided for use as a live biotherapeutic. As such, the invention extends to a live biotherapeutic comprising the strain of the invention. Also provided is use of the strain in killing or displacing or inhibiting or preventing colonisation of pathogenic gram positive bacteria (e.g. staphylococci or streptococci) on human skin or vagina. The present invention also extends to an isolated nucleotide sequence, or a fragment thereof, obtainable by culturing, or derived from, the strain of the invention, wherein the nucleotide sequence encodes for an amino acid sequence of the two-component lantibiotic of the invention.

The present invention further extends to an isolated polypeptide, or a fragment thereof, obtainable by culturing, or derived from, the strain of the invention, wherein the polypeptide forms at least part of the two-component lantibiotic of the invention.

According to a further aspect of the invention, there is provided a method for producing the two-component lantibiotic of the invention.

The method may comprise combining amino acid sequence SEQ. ID No. 2 or a variant amino acid sequence having at least 90% amino acid sequence identity thereto with at least one of:
- amino acid sequence SEQ. ID No. 4 or a variant amino acid sequence having at least 90% amino acid sequence identity thereto, and
- amino acid sequence SEQ. ID. No 6 or a variant amino acid sequence having at least 90% amino acid sequence identity thereto.

In certain embodiments, amino acid sequence SEQ. ID No. 2, or a variant amino acid sequence having at least 90% amino acid sequence identity thereto, is combined with both amino acid sequence SEQ. ID No. 4, or a variant amino acid sequence having at least 90% amino acid sequence identity thereto, and amino acid sequence SEQ. ID. No 6, or a variant amino acid sequence having at least 90% amino acid sequence identity thereto.

In certain embodiments, the amino acid sequences are produced using the nucleotide sequences of the invention. The two-component lantibiotic of the invention can be chemically synthesized using established methods.

Alternatively, the amino acid sequences may be isolated and purified from the strains of the invention which naturally produce the two-component lantibiotic or from a recombinant lantibiotic-producing bacteria (e.g., Lactococcus lactis or E. coli). In particular, the two-component lantibiotic of the invention can be produced by culturing naturally occurring lantibiotic-producing S. hominis strains of the invention or by culturing a lantibiotic-producing recombinant, and then isolating and/or purifying the lantibiotic using known techniques.

Accordingly, according to a further aspect of the invention, there is provided a method for producing the two-component lantibiotic of the invention, comprising cultivating at least one of the strains of the invention or a host cell comprising the vector of the invention under conditions suitable for the production of the two-component lantibiotic of the invention and optionally isolating or purifying the two-component lantibiotic of the invention from the strain. The host cell may be a bacterial cell and / or an insect cell and / or a yeast cell and / or a plant cell.

Precursor lantibiotic peptides are generally first modified and then become biologically active by proteolytic cleavage of the leader peptide from the mature peptide. The invention further provides amino acid sequences comprising precursor leader sequences and the active core peptides of SEQ ID No. 2, 4 and 6 respectively. The amino acid sequences of the leader "precursor" peptides are shown as SEQ ID No. 1 (HomA), 3 (homβ1) and 5 (homβ2). The complete amino acid sequences comprising both the precursor leader sequences and the active core peptides are shown as SEQ ID No. 31 (HomA), 32 (homβ1) and 33 (homβ2).

The invention further provides isolated nucleotide sequences encoding precursor leader sequences and the active core peptides of SEQ ID No. 2, 4 and 6 respectively. The nucleotide sequences encoding the leader "precursor" peptides are shown as SEQ ID No. 14 (HomA), 16 (homβ1) and 18 (homβ2). The complete nucleotide sequences encoding the precursor leader sequences and the active core peptides are shown as SEQ ID No. 34 (HomA), 35 (homβ1) and 36 (homβ2).

The invention further extends to an isolated lantibiotic modifying enzyme which effects dehydration and cyclization of a peptide or polypeptide and to an isolated nucleic acid molecule encoding same. In one embodiment, the modifying enzyme is homM1 having the amino acid sequence of SEQ ID No. 7. In another embodiment, the modifying enzyme is homM2 having the amino acid sequence of SEQ ID No. 8. Also provided is an isolated nucleotide sequence encoding for the modifying enzyme homM1 or homM2. The nucleotide sequence may comprise SEQ ID No. 20 (homM1) or 21 (homM2). Also provided is a vector comprising the nucleic acid molecule and a host cell comprising the vector.

The invention further extends to a method for modifying a peptide or polypeptide comprising contacting a peptide or polypeptide with a lantibiotic modifying enzyme, thereby effecting dehydration and cyclization of the peptide or polypeptide.

Enzymes that modify the lantibiotic peptides (homM1 and homM2) can be co-expressed by a recombinant host cell which expresses the alpha and beta peptides to provide in vivo modification of the peptides, or alternatively the modifying enzymes can be provided in an in vitro reconstitution reaction to modify the alpha and beta peptides. Accordingly, contact of a lantibiotic peptide with the modification enzymes specifically encompasses both in vivo and in vitro embodiments. The modification enzymes act upon an alpha or beta lantibiotic peptide so as to effect both at least one dehydration reaction and at least one cyclization reaction.

The invention further provides lantibiotic protease (homP) having the amino acid sequence of SEQ ID No. 9 and an isolated nucleotide sequence encoding for the protease. The nucleotide sequence is shown as SEQ ID No. 22.

Also provided are lantibiotic transporter (homT) having the amino acid sequence of SEQ ID No: 10 (ORF_T (46) ABC transporter BmrA), 11 (ORF_C (37) ABC transporter Yxlf), 12 (ORF_B (36) ABC transporter) or 13 (ORF_R (23) ABC transporter HisP regulator) and an isolated nucleotide sequence encoding for one or more of these sequences. The nucleotide sequences are shown as SEQ ID No. 23 (ORF_T (46) ABC transporter BmrA), 24 (ORF_C (37) ABC transporter Yxlf), 25 (ORF_B (36) ABC transporter) and 26 (ORF_R (23) ABC transporter HisP regulator).

The nucleic acid molecules of the invention can include at least the protein-coding portions of the HomA precursor peptide gene (SEQ ID No. 15) and at least one of the HomB1 (SEQ ID No. 17) and HomB2 (SEQ ID No. 19) precursor peptide genes. The nucleic acid molecules of the invention may further include the HomM1 (SEQ ID No. 20) and HomM2 (SEQ ID No. 21) modification enzyme genes. The nucleic acid molecules of the invention may further include one or any combination of, including all of, the HomP (SEQ ID No. 22), HomT (SEQ ID No. 23-26). The invention extends to nucleic acids comprising, consisting essentially of, or consisting of those specific sequences or a combination thereof.

The invention further provides a gene construct comprising or including one or more isolated nucleic acid molecules according to the invention and a control sequence, for example a promoter. There is further provided a vector including at least one of the nucleic acid molecules according to the invention. The vector may include a promoter which is operably linked to said nucleic acid molecule. Suitable vectors include viruses (e.g. Vaccinia virus, adenovirus, baculovirus etc), yeast vectors, phage, chromosomes, artificial chromosomes, plasmids or cosmid DNA. The vector may be a plasmid. The vector may be introduced into an organism such as Lactococcus lactis or E. coli. The invention extends to a host cell comprising the vector of the invention.

The nucleic acid molecules of the present invention can be expressed separately, i.e., inserted into separate vectors for expression and purification of individual gene products, namely alpha and beta peptides, modifying enzymes, protease and transporters, or alternatively collectively (e.g., as a gene cluster) inserted into a vector as an expression cassette.

The present invention further extends to a process for making an organism suitable for producing the two-component lantibiotic of the invention or a peptide thereof comprising the step of introducing an isolated nucleic acid molecule of the invention into the organism. In certain embodiments, the organism is Lactococcus lactis or E. coli.

The present invention further extends to a recombinant organism comprising an isolated nucleic acid molecule of the present invention. In certain embodiments, the organism is Lactococcus lactis or E. coli.

Suitably, the nucleic acid molecules of the present invention may be expressed to provide polypeptides.

According to a further aspect of the invention there is provided a method of producing a polypeptide encoded by a nucleic acid molecule of the invention, the method comprising the steps of:
(a) contacting a bacterial cell and / or an insect cell and / or a yeast cell and / or a plant cell with a vector as described herein, and
(b) cultivating said bacterial cell and / or insect cell and / or yeast cell and / or plant cell under conditions suitable for the production of the polypeptide.

Suitably the bacterial cell may be Lactococcus lactis or Escherichia coli. Suitably the polypeptide is encoded by a nucleotide sequence comprising SEQ ID No. 15, 17 or 19. In certain embodiments, the polypeptide is encoded by a nucleotide sequence derived from or obtainable by culturing S. hominis APC 3675 NCIMB 43358, S. hominis APC 2924 NCIMB 43356 or S. hominis APC 2925 NCIMB 43357.

The invention further provides a polypeptide produced substantially from the above method. As will be understood by those of skill in the art, such a polypeptide may be isolated or substantially purified from the mixture in which it is expressed. Suitably a polypeptide of the invention may be combined to form the two-component lantibiotic of the invention having anti-microbial activity.

According to a further aspect of the present invention, there is provided a method comprising contacting a precursor peptide containing the amino acid sequence for homA1 (SEQ ID No. 31) with a precursor peptide containing the amino acid sequence for homβ1 (SEQ ID No. 32) and/or homβ2 (SEQ ID No. 33. The method may further comprise contacting the precursor peptides with one or more of the two identified lantibiotic modification enzymes encoded by ORF38 (HomM1) and ORF42 (HomM2) capable of effecting dehydration and cyclization of the precursor peptide (SEQ ID No. 7 and 8), the lantibiotic protease (HomP) required to cleave the leader peptide from the precursor peptides (SEQ ID No. 9) and the ABC transporter-related peptides encoded by ORF23, ORF36 and ORF37 (SEQ ID No. 13, SEQ ID No. 12 and SEQ ID No. 11 respectively) and the peptide responsible for the transport of the novel antimicrobial encoded by ORF46 (SEQ ID No. 10).

The invention further extends to a kit for producing the two-component lantibiotic of the invention comprising precursor peptide HomA1 (SEQ ID No. 31) and at least one of precursor peptides HomB1 (SEQ ID No. 32) and/or HomB2 (SEQ ID No. 33) in combination with modifying enzymes HomM1 (SEQ ID No. 7) and HomM2 (SEQ ID No. 8) for producing the two-component lantibiotic of the invention. The kit may further comprise lantibiotic protease (homP) having the amino acid sequence of SEQ ID No. 9. The kit may further comprise the amino acid sequences for one or more of homT comprising the amino acid sequence of SEQ ID No. 10, 11, 12 and 13. The kit of the present invention may contain buffers suitable for carrying out dehydration and cyclization of the precursor peptides and/or an instruction manual.

The invention further provides a method of identifying putative lantibiotics comprising screening a genome sequence for the presence of a sequence having similarities to the LanM modification enzyme (e.g. homM1 or homM2). The method may be used to identify putative lantibiotic operons. Sequence similarity may be defined as having at least 30, 35, 40, 45, 50, 55, 60, 65, 70, 75, 80, 85, 90 or 95% sequence identity with a known LanM modification enzyme (e.g. homM1 or homM2).

### Brief Description of the Figures

The invention will be more clearly understood from the following description of some embodiments thereof, given by way of example only, with reference to the following figures in which:
Figure 1 shows: A) Antimicrobial producing isolate Staphylococcus hominis APC 3675 showing antimicrobial activity against Streptococcus agalactiae APC 1055. B) Spot assay of S. hominis APC 2924 against Lb. delbrueckii ssp. bulgaricus LMG 6901. C) Well diffusion assay of S. hominis APC 2925 against Lb. delbrueckii ssp. bulgaricus LMG 6901.
Figure 2 shows MALDI-TOF MS of active fractions displaying the masses of the peptides produced by Stapylococcus hominis APC 3675 (3396.4 Da - homA1 (Figure 2A); 3014.9 Da - homβ1; 3300.5 Da - homβ2 (Figure 2B)).
Figure 3 shows Lanthipeptides purified by RP-HPLC display antimicrobial activity and synergy in well diffusion assay against indicator organism L. delbrueckii subsp. bulgaricus LMG 6901.

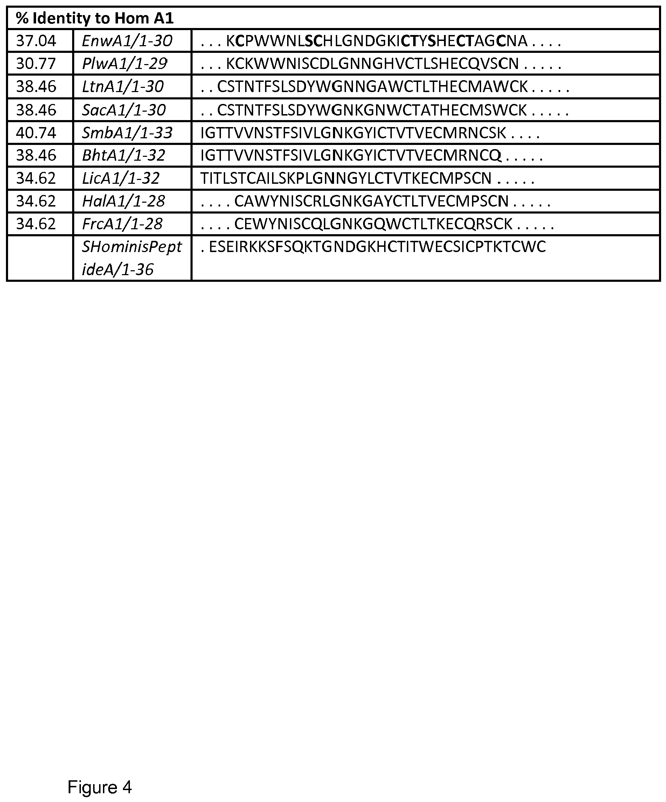

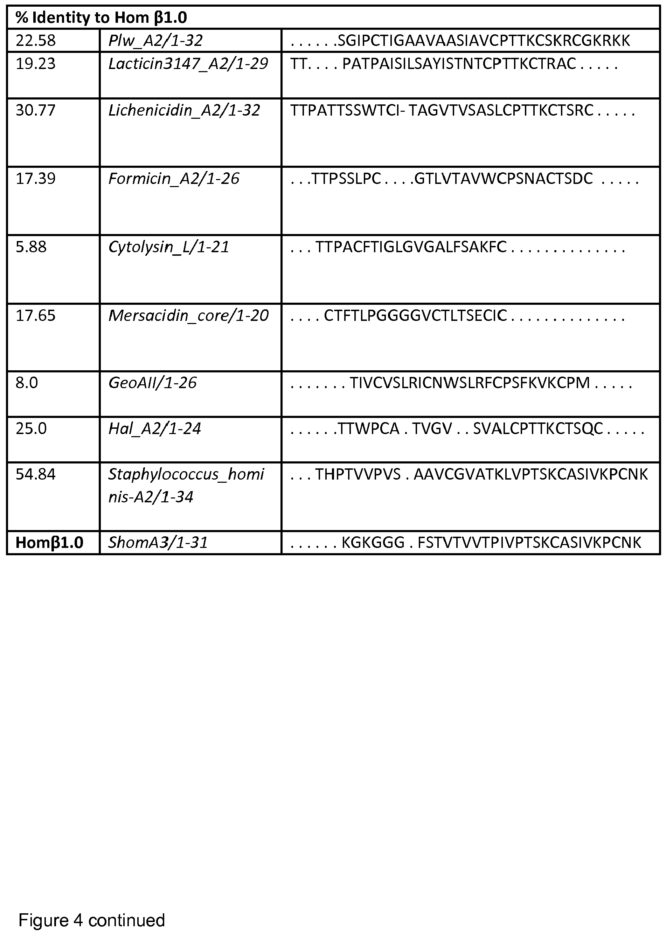

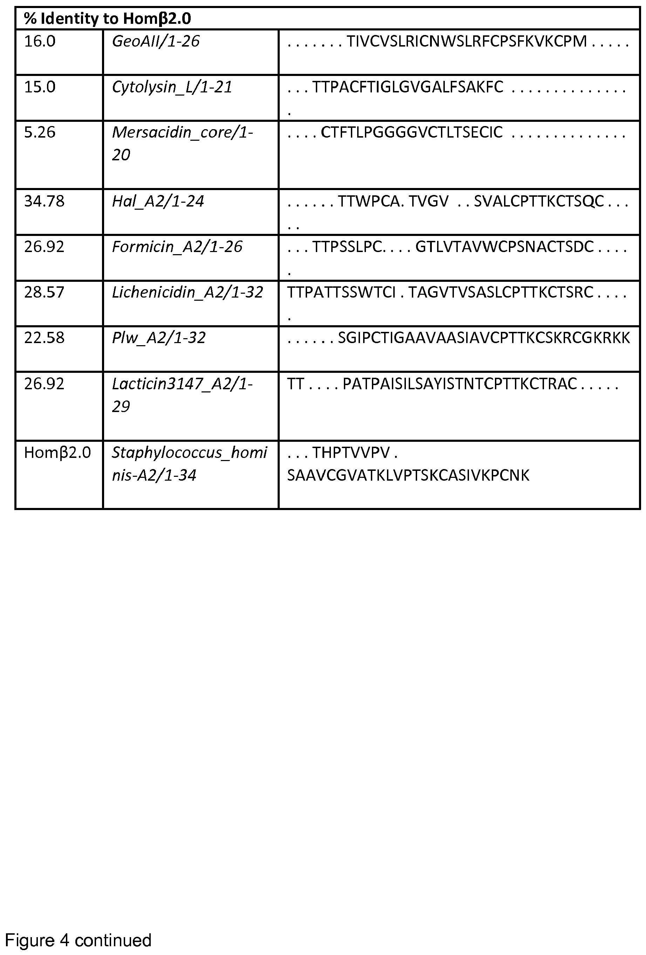

Figure 4 shows sequence alignment of homicin structural peptides. The desktop application Jalview was used to align amino acid residues calculated by Clustal Omega. The homicin peptides homA1 (α-peptide) (top image), homβ1 (middle image) and homβ2 (bottom image) were aligned against the previously described two-component bacteriocins. The percentage amino acid identities of each peptide with the homicin peptides are shown. The serine (S) residues, threonine (T) and cysteine (C) have been highlighted to indicate areas of homology and possible modifications in the structural peptides. The numbers at the bottom of image represent the predicted dehydrations for each structural peptide.
Figure 5 shows predicted two-component lantibiotic operon representation of bagel4 output of the three S. hominis strains: APC 3675, APC 2924 and APC 2925. The homicin operon appears to contain one gene encoding the Hom α-peptide and two genes encoding homβ1 and homβ2 peptides. HomM1 (left - orf M1) and homM2 (right - orf M2) encode the accompanying modification enzymes. HomT and homP are involved in bacteriocin transport and leader cleavage, respectively.
Figure 6 shows Homicin structural prediction. The structures of the homicin α and β peptides were predicted using the Ltnα and Halα structures and the Halβ and Lichβ structures as templates. The conservation of key amino acids suggests a structural homology between the peptides. The rings formed from lanthionine and methyllanthionine bridges are labelled alphabetically and Ser/Thr dehydrations highlighted.
Figure 7 shows the BLAST Ring Image Generator (BRIG) used to visually compare the S. hominis, Homicin operon, present within the three draft genomes. S. hominis APC 3675 was used as a reference sequence due to the higher degree of coverage from the sequencing platform.
Figure 8 shows biosynthetic gene cluster detected by antiSMASH in the 3 S. hominis strains APC 3675, APC 2924 and APC 2925 with 75% similarity to a prevalent siderophore, staphyloferrin A.
Figure 9 shows PFGE profile of 3 S. hominis strains APC 2925, APC 2924 and APC 3675.
Figure 10 shows differences in gene composition between the 3 S. hominis strains on contig containing homicin operon - the same colour represents the same amino acid sequence of gene.

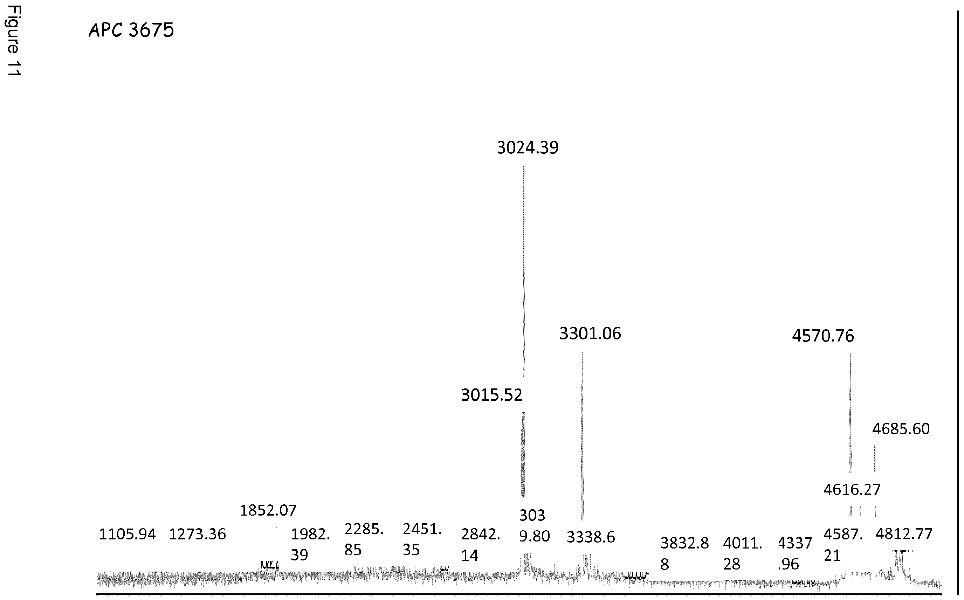

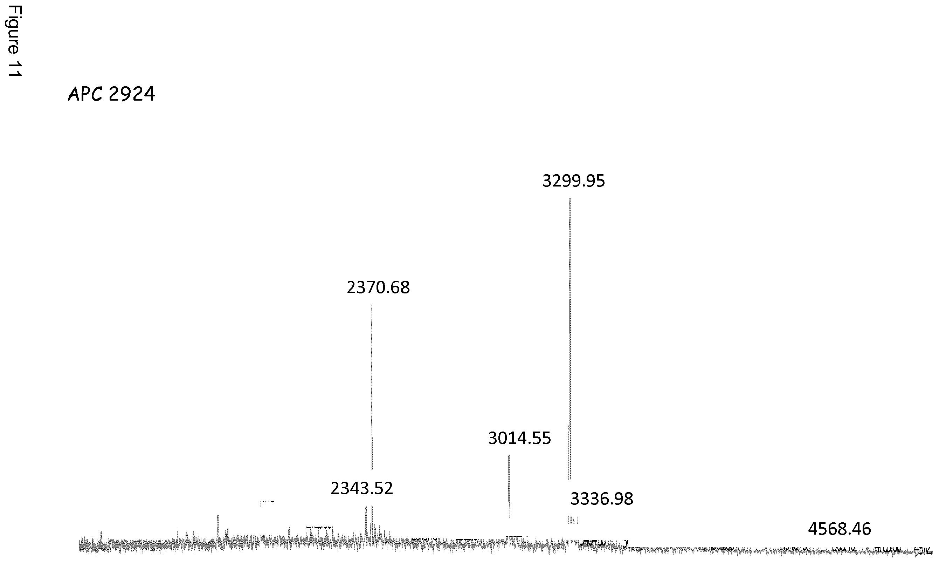

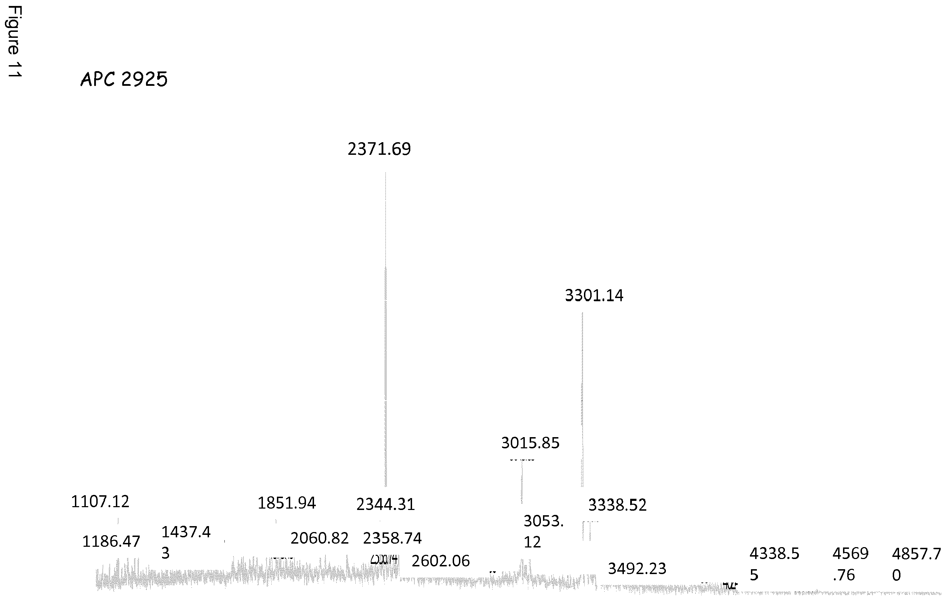

Figure 11 shows that colony mass spec results of the 3 S. hominis strains again shows a difference between APC 3675 (vaginal isolate) to APC 2924 and APC 2925 (skin isolates) with the skin isolates showing an extra peak on the left hand side at ∼2370Da.
Figure 12 shows that the colour difference observed in cell preparation of S. hominis strain APC 3675 (far left of image) and S. hominis strains APC 2924, APC 2925 prior to purification is quite obvious, suggesting APC 3675 is different to the other 2 S. hominis strains in this study.

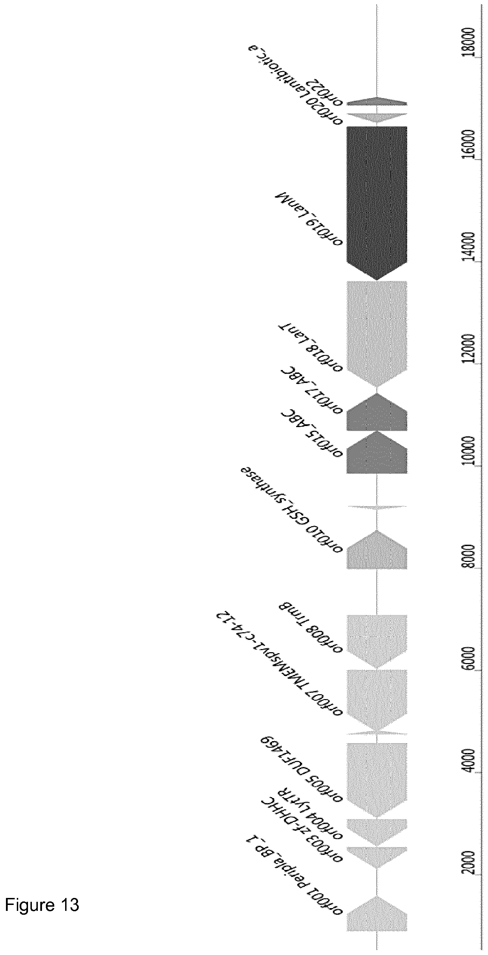

Figure 13 shows Bagel3 identification of a putative lantibiotic operon in V. dokdonesis DSW-10. S. hominis APC 3675 HomM modification enzymes were found to be 38% identical to the LanM displayed here in the putative operon. The structural peptides identified above for ORF20 and ORF22 have 42% and 38% identity to haloduracin and lichenicidenVK21 α-peptides. This putative operon has not previously been published in the literature for V. dokdonesis DSW-10.

### Detailed Description of the Invention

The two-component lantibiotic of the invention can be formed from precursor peptides (66-amino-acid α-peptide (SEQ ID No. 31), 54-amino-acid β-peptides, homβ1 (SEQ ID No. 32) and/or homβ2 (SEQ ID No. 33)) that are ribosomally or artificially synthesised. The α-peptide may comprise a lanthionine bridge between Ser-11 and Cys-21. Additionally or alternatively, the α-peptide may comprise methyllanthionine bridges between one or more of Thr-22 and Cys-27, Thr-24 and Cys-30, Thr-32 and Cys-35, and Thr-34 and Cys-37. Ser-9 may be dehydrated to its respective Dha residue. Ser-28 may remain unaltered (Figures 4 and 6). The first β peptide may comprise bridges between Thr-26 and Cys-29, and Ser-31 and Cys-36. This would result in Ser-15, Thr-16, and Thr-21 being dehydrated to their respective Dha and Dhb residues, whilst Thr-18 and Ser-27 remain unaltered (Figures 4 and 6). The second β peptide may comprise bridges between Ser-13 and Cys-17, Thr-26 and Cys-29, and Ser-31 and Cys-36. This would result in Thr-4, Thr-7 and Thr-21 being dehydrated to their respective Dha and Dhb residues, whilst Ser-27 would remain unaltered (Figures 4 and 6).

In relation to sequences provided by the invention, sequence identity may be determined using a suitable mathematical algorithm. Computer implementations of such mathematical algorithms can be utilized for comparison of sequences to determine sequence identity. Such implementations include, but are not limited to, CLUSTAL in the PC/Gene program (available from Intelligenetics, Mountain View, California), the ALIGN program (Version 2.0) and GAP, BESTFIT, BLAST, FASTA and TFASTA in the Wisconsin Genetics Software Package, Version 8 (available from Genetics Computer Group (GCG), 575 Science Drive, Madison, Wisconsin, USA)). Suitably alignments using these programs may be performed using the default parameters.

As used herein, "sequence identity" or "identity" in the context of two nucleotide or polypeptide sequences makes reference to a specified percentage of residues in the two sequences that are identical when aligned for maximum correspondence over a specified comparison window, as measured by sequence comparison algorithms or by visual inspection. Suitably, a specified comparison window is selected from a sequence encoding or representing at least 20, at least 25, at least 30, at least 40, at least 50, at least 75, at least 80, at least 85, at least 90, at least 95, at least 100, at least 105, at least 110, at least 115, at least 120, at least 121, at least 122, at least 123, at least 150, at least 200, at least 250 or most preferably all of the amino acids of a specified polypeptide being aligned. In certain embodiments, the specified comparison window is all of the residues of the sequences. When percentage of sequence identity is used in reference to proteins it will be understood by those of skill in the art that residue positions which are not identical often differ by conservative amino acid substitutions, i.e. wherein amino acids are substituted with amino acids which have similar chemical properties to those amino acids which are replaced. The percent sequence identity may be adjusted upwards to correct for the conservative nature of a substitution.

Amino acids may be grouped according to the properties of their side chains, for examples as follows: (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); and (4) basic: Lys (K), Arg (R), His(H). Alternatively, naturally occurring residues may be divided into groups based on common side-chain properties, for example: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe. Conservative substitutions entail exchanging a member of one of these classes for another member of the same class, that is an amino acid residue with side chains having similar biochemical properties to the amino acid residue being substituted. Preferably when the amino acid sequences of the invention are modified by way of conservative substitution of any of the amino acid residues contained therein, these changes have no effect on the functional activity of the resulting polypeptide when compared to the unmodified polypeptide. The effect (if any) on function of a change in an amino acid residue may be easily assessed by a person skilled in the art, for example, by generating a mutation library, screening for changes in function and sequencing the mutants.

Hybridisation refers to the binding, duplexing or hybridizing of a molecule only to a particular nucleotide sequence under stringent conditions when that sequence is present in a complex mixture (e.g., total cellular) DNA or RNA. Stringent hybridisation occurs when a nucleic acid binds the target nucleic acid with minimal background. Typically, to achieve stringent hybridisation, temperatures of around 1°C to about 20°C, more preferably 5°C to about 20°C below the Tm (melting temperature at which half the molecules dissociate from their partner) are used. However, it is further defined by ionic strength and pH of the solution.

The stringent conditions may be selected from highly stringent conditions, medium stringent conditions, stringent conditions and low stringent conditions. An example of highly stringent wash conditions is 0.15 M NaCl at 72 C for about 15 minutes. An example of a stringent wash condition is a 0.2X SSC wash at 65°C for 15 minutes (see, Sambrook and Russell, infra, for a description of SSC buffer). Often, a high stringency wash is preceded by a low stringency wash to remove background probe signal. An example of a medium stringency wash for a duplex of, for example, more than 100 nucleotides, is 1X SSC at 45°C for 15 minutes. An example of a low stringency wash for a duplex of, for example, more than 100 nucleotides, is 4-6X SSC at 40°C for 15 minutes. For short probes (for example about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long probes (for example, > 50 nucleotides).

Modifications to the nucleic acids of the present invention are also contemplated as long as the essential structure and function of the peptide or polypeptide encoded by the nucleic acids are maintained. Nucleotide sequences may be codon-optimised or otherwise modified to increase the efficiency of expression of the polypeptides.

Staphylococcus hominis is a coagulase-negative member of the bacterial genus Staphylococcus, consisting of Gram-positive, spherical cells in clusters. Colonies of S. hominis are small, usually 1-2 mm in diameter after 24 hours' incubation at 35°C, and white or tan in colour.

The S. hominis strains were deposited with NCIMB Ltd, Ferguson Building, Craibstone Estate, Bucksburn, Aberdeen, AB21 9YA, Scotland, UK by University College Cork - National University of Ireland, Cork, College Road, Cork, Ireland on 7 February 2019 and assigned the accession numbers NCIMB 43358 (APC 3675), 43356 (APC 2924) and 43357 (APC 2925).

It is understood that the strain of the present invention is not limited to the deposited strain since mutants and variants of the strain, for example, cell fusion strains or recombinant bacteria strains, may also be used in the process of the invention. The term "mutant" is understood herein to refer to a microorganism which is derived from the deposited strain by one or more mutations. The mutant should retain the desired anti-microbial activity of the deposited strain or have improved anti-microbial activity over that of the deposited strain. These mutants can be related bacteria isolates that have either arisen spontaneously or under selection conditions designed to isolate the mutants. For example, commercial kits are available that generate random mutations following which all generated mutants can be screened for anti-microbial activity. The term "variant" is understood herein to refer to a microorganism which comprises the anti-microbial activity of the deposited strain. The variant should retain the desired anti-microbial activity of the deposited strain or have improved anti-microbial activity over that of the deposited strain. These variants can be related bacteria isolates that have either arisen spontaneously or under selection conditions designed to isolate the variants. The variants can also be recombinant bacteria which express the two-component lantibiotic or a variant thereof and which can be constructed using genetic engineering methods which would be well known to those skilled in the art. All generated variants may be easily screened for anti-microbial activity.

In certain embodiments, the deposited strain is manipulated using genetic engineering methods such that genes responsible for antibiotic resistance are not expressed. For example, strain APC 3675 may be manipulated such that genes responsible for beta-lactam resistance, macrolide resistance, streptogramin A resistance and tetracycline resistance are not expressed.

In certain embodiments, the bacterial infection is infection by a gram positive bacteria. In certain embodiments, the bacterial infection is infection by bacteria selected from one or more of the group consisting of streptococci, staphylococci and lactobacilli species. In certain embodiments, the streptococci species is selected from one or more of the group consisting of Streptococcus agalactiae, Streptococcus bovis and Streptococcus dysgalactiae. In certain embodiments, the lactobacilli species is selected from one or more of the group consisting of Lactobacillus farraginis, Lactobacillus delbrueckii subsp. Bulgaricus.

In certain embodiments, the streptococci species is Streptococcus agalactiae. Accordingly, the invention extends to the treatment or prevention of infection by Group B Streptococcus. The subject in need of treatment may be a pregnant female or an infant. The two-component lantibiotic or the strain of the invention may be administered prophylactically to prevent GBS colonisation in a pregnant female and/or an infant or may be administered to treat GBS infection in a pregnant female and/or an infant. The administration of Homicin may have potential as a prophylactic to limit GBS vaginal colonisation during pregnancy and thus prevent mother-infant transmission of this infectious organism which can cause significant morbidity and mortality in infected infants.

In certain embodiments, the bacterial infection is infection by a skin pathogen, such as Corynebacterium xerosis. Accordingly, the invention extends to the treatment or prevention of pneumonia, sepsis and conjunctivitis associated with skin pathogens, such as Corynebacterium xerosis. The subject in need of treatment may be an immunocompromised individual.

Anti-microbial activity may be assayed using methods which would be well known to persons skilled in the art, for example, spot assays and well diffusion assays as described in the below examples.

Treatment (i.e. the two-component lantibiotic or the strain) may be combined with one or more standard treatments for the disease or condition in question. The treatment may be administered alone or may be administered as a pharmaceutical composition which will generally comprise a suitable pharmaceutically acceptable excipient, diluent or carrier. The pharmaceutically acceptable excipient, diluent or carrier may be selected depending on the intended route of administration. Examples of suitable pharmaceutical carriers include water, glycerol and ethanol. The two-component lantibiotic of the invention may be administered as a combined peptide formulation.

The treatment may be administered to a subject in need of treatment via any suitable route. In particular, the treatment may be administered orally or topically as it is capable of crossing the blood-brain barrier. The treatment may also be administered parenterally by injection or infusion. Examples of preferred routes for parenteral administration include, but are not limited to, intravenous, intracardial, intraarterial, intraperitoneal, intramuscular, intracavity, subcutaneous, transmucosal, inhalation and transdermal. Routes of administration may further include topical and enteral, for example, mucosal (including pulmonary), oral, nasal and rectal. The treatment may also be administered via nanoparticles, microspheres, liposomes, other microparticulate delivery systems or sustained release formulations placed in certain tissues including blood. Typically, the two-component lantibiotic of the invention may be provided in the form of a vaginal suppository, for example, for administration when used as an antimicrobial against Group B Streptococcus, or as a skin cream, for example, when used against skin pathogens, such as Corynebacterium xerosis.

The treatment is typically administered to a subject in a "therapeutically effective amount", this being an amount sufficient to show benefit to the subject to whom the treatment is administered. The actual dose administered, and rate and time-course of administration, will depend on, and can be determined with due reference to, the nature and severity of the condition which is being treated, as well as factors such as the age, sex and weight of the subject being treated, as well as the route of administration. Further due consideration should be given to the properties of the treatment, for example, its in-vivo plasma life and concentration in the formulation, as well as the route, site and rate of delivery. Prescription of treatment, e.g. decisions on dosage, etc., is ultimately within the responsibility and at the discretion of general practitioners and other medical doctors, and typically takes account of the disorder to be treated, the condition of the individual patient, the site of delivery, the method of administration and other factors known to practitioners.

Dosage regimens can include a single administration, or multiple administrative doses. The treatment can further be administered simultaneously, sequentially or separately with other therapeutics and medicaments which are used for the treatment of the disease or condition for which the treatment is being administered.

### Definitions

Unless otherwise defined, all technical and scientific terms used herein have the meaning commonly understood by a person who is skilled in the art in the field of the present invention.

Typically, the terms "subject" and "patient" are used interchangeably herein. The subject is typically a mammal, more typically a human.

The term "treatment" as used herein and associated terms such as "treat" and "treating" means the reduction or inhibition of the progression, severity and/or duration of the bacterial infection or at least one symptom thereof. The term 'treatment' therefore refers to any regimen that can benefit a subject. Treatment may include curative, alleviative or prophylactic effects.

A peptide, polypeptide, protein, bacteria or nucleic acid molecule of the invention may be provided in an "isolated" form. The term "isolated" as used herein means that the substance is provided in a form that differs from its naturally occurring environment. It may be used to refer to an in vitro preparation, isolation and/or purification of a peptide, polypeptide, protein, bacteria or nucleic acid molecule of the invention, such that it is not associated with in vivo substances or is substantially purified from in vivo substances or is present outside its naturally occurring environment.

As used herein, an "isolated lantibiotic" is one which has been identified and separated and/or recovered from a component of its natural environment. "Synthetic lantibiotics" or "recombinant lantibiotics" are generally generated using recombinant technology or using peptide synthetic techniques known to those of skill in the art.

An "isolated" bacteria is also one which has been identified and separated and/or recovered from a component of its natural environment.

As used herein, a polypeptide can be considered to be "derived from" a strain if the polypeptide originates directly or indirectly from the strain. The polypeptide may be encoded by a part of the genome of the strain and may therefore be obtainable directly from culturing the strain, including for example being expressed in the strain, for example in microbial whole cells, being present in the cytosol thereof, being present in a cell culture thereof or being present in a cell lysate thereof. The polypeptide may also be synthetically prepared from a gene which is endogenous to the strain of the invention following isolation of the gene from the strain and sequencing of the gene. For example, the polypeptide may be synthetically prepared, and/or be obtained using recombinant DNA technology, such as from a genetically engineered plasmid/host cell system in which the plasmid includes a nucleic acid polymer which encodes the polypeptide.

As used herein the terms "nucleic acid" or "nucleotide sequence" includes genomic DNA, cDNA or RNA.

The phrase "consists essentially of" or "consisting essentially of" as used herein means that a polypeptide or nucleotide sequence may have additional features or elements beyond those described provided that such additional features or elements do not materially affect the ability of the polypeptide to metabolise nitriles. For example, a polypeptide consisting essentially of a specified sequence may contain one, two or three additional, deleted or substituted amino acids, at either end or at both ends of the sequence provided that these amino acids do not interfere with its function. Similarly, a polypeptide of the invention may be chemically modified with one or more functional groups provided that such functional groups do not interfere with its function.

The terms "polypeptide", "peptide", or "protein" are used interchangeably herein to designate a linear series of amino acid residues connected one to the other by peptide bonds between the alpha-amino and carboxy groups of adjacent residues. The amino acid residues are usually in the natural "L" isomeric form. However, residues in the "D" isomeric form can be substituted for any L-amino acid residue, as long as the desired functional property is retained by the polypeptide.

A peptide, polypeptide, protein, bacteria or nucleic acid molecule of the invention may be provided in a "purified" form. As used herein, the term "purified" refers to a degree of separation that is higher than isolated. A nucleic acid or polypeptide of the presently disclosed subject matter is purified if it is substantially free of cellular material, viral material, or culture medium when produced by recombinant DNA techniques, or chemical precursors or other chemicals when chemically synthesised.

The words "comprises/comprising" and the words "having/including" when used herein with reference to the present invention are used to specify the presence of stated features, integers, steps or components, but do not preclude the presence or addition of one or more other features, integers, steps, components or groups thereof.

As used herein, terms such as "a", "an" and "the" include singular and plural referents unless the context clearly demands otherwise.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### Examples

### Materials and Methods

### Patient recruitment and sampling

Approval for collection of the vaginal swabs was obtained by the Cork Research Ethics Committee of the Cork Teaching Hospitals, and informed consent was provided by each of the women taking part in the study. In total 130 women were recruited at the colposcopy clinic St. Finbarr's Hospital, Cork, of which 125 swabs were collected (5 swabs became unobtainable at the time of collection). A high vaginal swab was obtained from each woman who underwent a colposcopy at the clinic, with the exception of women on antibiotics or probiotic treatment. The sample of vaginal fluid was collected using a Dacron swab (Medical Packaging Swab-Pak™, Camarillo, CA, USA). The swabs were stored in a cool box at 4 °C and collected from the clinic within 24 hours. Skin swabs were obtained from healthy volunteers in a study by O'Sullivan et al., 2018. In total, 20 skin swabs were collected from 13 females and 7 males. Approval to obtain skin swabs for this study was acquired from the Cork Clinical Research Ethics Committee under Protocol number APC071 and informed consent was granted by all 20 volunteers.

### Isolation of bacteria from vaginal and skin swabs

Vaginal swabs were incubated aerobically at 37°C for 24 hours (h) in 5 mL of GBS selective enrichment broth. Enrichment broth contained the following ingredients: Todd Hewitt broth (30 g/L), yeast extract (10 g/L) and nalidix acid (15 mg/L). After incubation, 1 mL of the enrichment broth was serially diluted in maximum recovery diluents (MRD; Oxoid Ltd., Basingstoke, Hampshire, UK) and plated on brain heart infusion (BHI) agar (Merck); incubated aerobically at 37°C for 24 h. Bacteria were isolated from skin swabs as described by O'Sullivan et al., 2018. riefly, each swab (Sarstedt swabs Amies, PS, Viscose) was dipped in sterile saline prior to swabbing the body area. Swabs were vortexed in 2 mL of MRD (Oxoid), serial dilutions were carried out in MRD (tenfold dilutions) and 100 µL of the dilutions were spread plated onto Mannitol Salt Agar (MSA; Oxoid Ltd.) and Brain Heart Infusion (BHI) agar (Merck, Darmstadt, Germany) and incubated aerobically at 37°C for 48h.

### Identification of antimicrobial producing isolates

The antimicrobial producing isolates were identified by 16S rRNA sequencing using universal primers, Uni-F (5'-AGAGTTTGATCCTGGCTCAGG-3') (SEQ ID No. 27) and UniR (5'-ACGGCAACCTTGTTACGAGT-3') (SEQ ID No. 28). PCR product was cleaned using the GenElute™ PCR Clean-Up Kit (Sigma) and subsequent PCR product was sequenced by Genewiz (Essex, UK). For species verification, degenerate primers for amplification of the dnaJ gene (forward primer: 5'-GCCAAAAGAGACTATTATGA-3' (SEQ ID No. 29) and reverse primer: 5'-ATTGYTTACCYGTTTGTGTACC-3') (SEQ ID No. 30) were employed for the PCR reactions using conditions outlined by (Shah et al., 2007).

### Detection of antimicrobial activity

A) Spot assays: To detect antimicrobial activity from the vaginal isolates, colonies isolated on BHI agar (from vaginal swabs), were overlaid with 'sloppy' BHI agar (0.75% agar) inoculated with 0.5% of S. agalactiae APC 1055. For vaginal isolates, Streptococcus agalactiae APC 1055 was used as an indicator microorganism for the isolation of bacteria with antimicrobial activity against GBS. Of the 125 vaginal swabs, only one swab was found to contain isolates which displayed a zone of inhibition against the indicator organism. Subsequently, the identified antimicrobial producing isolate, S. hominis APC 3675, was further characterised and tested against a range of indicator organisms (Table 1). This involved initially spotting 10 µL of the S. hominis APC 3675 culture onto BHI agar plates, which were incubated overnight and subsequently overlaid with 10 mL of the appropriate agar seeded at 0.5% with the indicator organism (Table 1). A similar approach was adopted to detect antimicrobial activity from skin isolates (O'Sullivan et al., 2018). Lactobacillus delbrueckii ssp. bulgaricus LMG 6901 was used as the primary indicator microorganism to detect antimicrobial activity from skin isolates and colonies of different morphology were isolated from BHI and MSA plates. Skin isolates obtained from BHI plates were overlaid with L. delbrueckii ssp. bulgaricus and skin isolates obtained from MSA plates were overlaid with either Listeria innocua DPC 3572 or Methicillin-Resistant Staphylococcus aureus (MRSA) DPC 5645. S. hominis skin isolates APC 2924 and APC 2925 were isolated from the toe web-space area and were isolated from BHI plates.
B) Well diffusion assays (WDA): Following spot assays, WDA were then carried out on cultures that produced a zone in the spot assays. Cell free supernatants (CFS) were obtained by centrifuging 10 mL overnight cultures (in Sorvall Legend RT) at 4000xg for 20 minutes (Ryan, Rea, Hill, & Ross, 1996). 50µl of neutralised CFS was obtained from each of the three S. hominis strains and used in WDA containing 0.25 % inoculum of L. delbrueckii ssp. bulgaricus LMG 6901 and GBS among other targets (Table 1).

### Preparation of S. hominis APC 3675, APC 2924 and APC 2925 for peptide purification

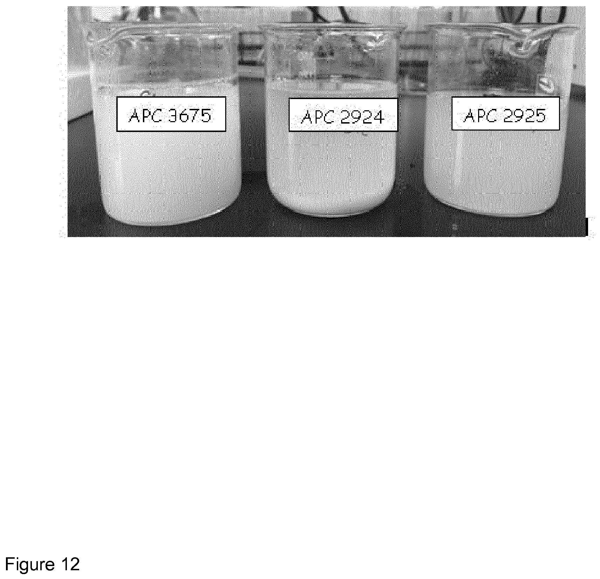

5 mL volumes of S. hominis APC 3675, S. hominis APC 2924 and S. hominis APC 2925 were incubated overnight and used to inoculate 20 mL (1% inoculum) of BHI broth, incubated by shaking aerobically at 180 rpm for 8 hours at 37 °C. Subsequently, 400 mL of BHI XAD was inoculated. BHI XAD is prepared by passing BHI media through a column containing XAD beads prior to autoclaving. Cells were separated from supernatant and the cell pellet mixed with approximately 100 mL 70% propan-2-ol 0.1% TFA (IPA). The suspension was stirred for 3 hours at room temperature and re-centrifuged. See Figure 12 for image of the cell suspensions showing differences in cell preparation between the cultures.

### Purification of antimicrobial peptides produced by S. hominis APC 3675, APC 2924 and APC 2925

Supernatant was collected from each of the three cultures and IPA evaporated. The resulting samples were applied to a 12 mL 2 g C18 SPE column (Phenomenex, Cheshire, UK) pre-equilibrated with methanol and water. The column was washed with 15 mL 30% ethanol (30E) and antimicrobial activity eluted with 20 mL IPA. Eluents were assayed on a Lactococcus lactis HP indicator plate to show that activity was retained by the column.

### HPLC analysis of Cell Extracts

Samples were concentrated and applied to a semi preparative Proteo Jupiter (10 x 250 mm, 90Å, 4µ) RP-HPLC column (Phenomenex, Cheshire, UK) running a 25-45% acetonitrile 0.1% TFA where buffer B is 100% acetonitrile 0.1% TFA. Eluent was monitored at 214 nm and fractions were collected at 1 minute intervals. Fractions were assayed on Lactococcus lactis HP indicator plates.

### Mass spectrometry (MS)

MALDI-TOF MS (Axima TOF2 MALDI-TOF MS, Shimadzu Biotech) was performed to determine the molecular mass of the antimicrobial peptides. The masses detected were compared to those identified in the bacteriocin operon of S. hominis APC 3675, APC 2924 and APC 2925 (described below). For partially purified fractions an additional run on the RP-HPLC column was performed on a 30-35% acetonitrile gradient where buffer B is 90% acetonitrile 0.1% TFA. Eluent was monitored at 214 nm and fractions were collected at 1-minute intervals. Active fractions were confirmed by agar well diffusion assay and lyophilised for further peptide stability and minimal inhibitory concentration (MIC) assays.

### Colony Mass Spectrometry

Colonies from three S. hominis strains APC 3675, APC 2924 and APC 2925 were mixed with 50 µL propan-2-ol 0.1% TFA, vortexed three times and centrifuged at 16000xg for 30 seconds. MALDI TOF mass spectrometry was performed on the CFS using an Axima TOF2 MALDI TOF mass spectrometer (Shimadzu Biotech, Manchester, UK). An aliquot (0.5 µL) of matrix solution (a-cyano 4-hydroxy cinnamic acid, 10 mg ml-1 in acetonitrile-0.1% (v/v) trifluoroacetic acid) was deposited onto the target and left for 10 seconds before being removed. The residual solution was allowed to air-dry and 0.5 µL of sample solution was deposited onto the pre-coated sample spot; 0.5 µL of matrix solution was added to the deposited sample and allowed to air-dry. The sample was then analysed in positive-ion linear mode and peptide masses compared to the bacteriocin database, 'Bactibase' to identify putative bacteriocins.

### Peptide stability

To confirm if the antimicrobial activity was due to the production of a bacteriocin, the partially purified peptides obtained from each of the three S. hominis isolates were treated with proteinase K and α-chymotrypsin (10 mg/mL, Sigma-Aldrich). The peptide mixture containing both α and β peptides was incubated at 37 °C for 3 h with each protease, followed by treatment at 100 °C for 15-20 min to inactivate these proteases. The sample was then tested for antimicrobial activity in well diffusion assay against the indicator S. agalactiae APC 1055 and L. delbrueckii ssp. bulgaricus DPC 6901.

### Minimal inhibitory concentration (MIC) assay

After partial purification of the identified lantipeptides, fractions containing α and β peptides were lyophilized and diluted separately in sterile water to a concentration of 1 mM. The peptide preparation of APC 3675 was finally diluted in BHI broth to give the desired starting concentrations (in a 1:1 ratio), and two-fold serial dilutions were performed in 96-well microtitre plates containing approximately 1 x 105 cfu/mL of S. agalactiae APC 1055. MIC readings were taken in triplicate after 18 h incubation at 37°C and MIC was recorded as the lowest concentration of the antimicrobial at which there was no visible growth of the indicator organism.

### Draft Genome Sequencing

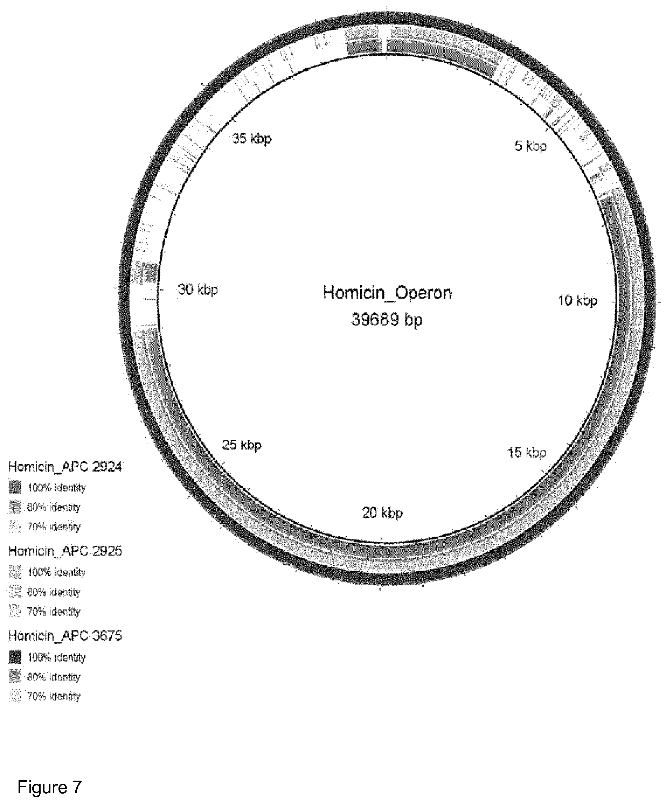

Genomic DNA was extracted using the GenElute bacterial genomic kit (Sigma-Aldrich) and the Nextera XT DNA kit (Illumina) was used for library preparation. The DNA was quantified using a Qubit 3.0 fluorometer. Sequencing was performed using Illumina's MiSeq platform using paired-end 2 x 300 base pair reads in the Teagasc Sequencing Centre, Teagasc Food Research Centre Moorepark. Reads for APC 3675 were assembled de novo, using SPAdes (version 3.10.0), resulting in 152 total contigs, including 39 large contigs (>500bp). ORFs were identified and annotated using Prokka (version 1.11). Further manual annotation was implemented with ARTEMIS, and the BLAST Ring Image Generator (BRIG) was used to visually compare the contig containing the bacteriocin operon in each of the three S. hominis draft genomes (Figure 7).

### In silico validation of bacteriocin operon

To identify a potential bacteriocin cassette in the three draft genomes of interest, Bagel4 was used (Van Heel et al., 2018). Bagel4 (http://bagel4.molgenrug.nl/), also known as the BActeriocin GEnome mining tool (BAGEL), is an online tool used for the identification of genes encoding bacteriocins and RiPPs. Lanthipeptides, sactipeptides, glycocins and others can be detected using this tool. In addition, antiSMASH3.0 was used to detect genes encoding biosynthetic pathways responsible for the production of secondary metabolites, such as lantipeptides (Weber T et al., 2015). To perform multiple sequence alignments of nucleotide and amino acid residues, Clustal Omega was used in conjunction with the desktop application Jalview-2 (Waterhouse et al., 2009, Sievers et al., 2011). To identify plasmid constructs within the annotated contigs, the online tool PlasmidFinder-1.3 was used (Carattoli et al., 2014) and to identify virulence genes and genes responsible for antimicrobial resistance, VirulenceFinder-1.5 and ResFinder-3.0 were used, respectively (Joensen et al., 2014, Zankari et al., 2012).

### Pulsed Field Gel Electrophoresis (PFGE)

To compare the genetic fingerprints of the S. hominis strains, APC 3675, APC 2924 and APC 2925, PFGE was carried out on all three strains following the method described by Bannerman et al. with minor deviations as described by O'Sullivan et al., 2018. To optimise the procedure, additional washing steps were carried out for 20 minutes (mins) with TE 10/0.1 buffer, followed by 10 mins in buffer solution and digestion with Sma1 enzyme.

### Results

### Isolation of S. hominis APC 3675

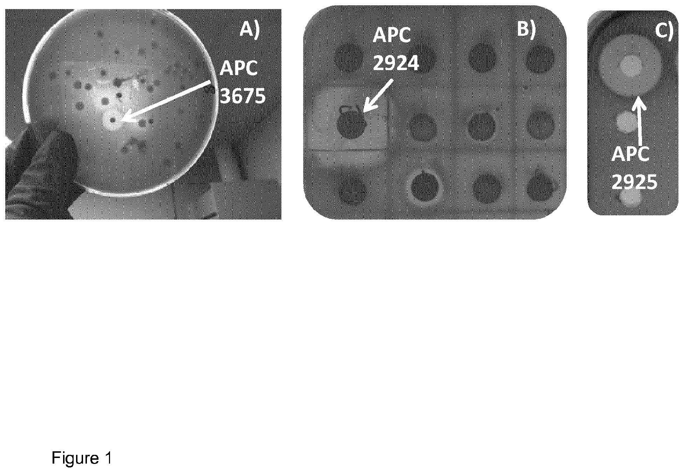

S. hominis APC 3675 was isolated from the vaginal microbiota of a healthy female attending a colposcopy clinic in Cork, Ireland. In an initial screen for bacteriocin producers, the strain was found to inhibit S. agalactiae APC1055 in an overlay assay (Figure 1). Interestingly, no zone of inhibition was detected in CFS, initially indicating that the antimicrobial substance is cell associated. However, on shaking antimicrobial activity was observed in the CFS.

The three homicin producers (APC 3675, APC 2924 and APC 2925) displayed inhibitory activity with a 2-3mm zone of inhibition against twelve of 33 strains tested in spot assays (see Table 1). S. hominis isolates were subsequently screened by WDA to determine the spectrum of inhibition in CFS (Table 1). Overall, homicin exhibited a relatively narrow spectrum of activity inhibiting streptococci, staphylococci and lactobacilli species, with desired antimicrobial activity against S. agalactiae spp.

**Table 1. Growth conditions of indicator strains and inhibition spectrum of the three S. hominis APC 3675, APC 2924 and APC 2925 following spot plate and well diffusion assays. MRS, de Man, Rogosa and Sharpe; BHI, brain-heart infusion. -, No activity; +, 0.5-1.5mm inhibition zone; ++, 2-3.5mm inhibition zone.**

| **Indicator strain** | **strain number** | **Temp.(°C)** | **conditions** | **media** | **zone** |
|---|---|---|---|---|---|
| *Bacillus subtilis* | DPC 3344 | 37 | Aerobic | BHI | + |
| *Bacillus subtilis* | LMG | 37 | Aerobic | BHI | - |
| *Bacillus cereus* | DPC 6085 | 37 | Aerobic | BHI | - |
| *Bacillus paralicheniformis* | APC1576 | 37 | Aerobic | BHI | - |
| *Bacillus licheniformis* | DSM 13 | 37 | Aerobic | BHI | - |
| *Corynebacterium xerosis* | DPC5629 | 37 | Aerobic | BHI | ++ |
| *Cutibacterium acnes* | LMG 16711 | 37 | Anaerobic | SLA | - |
| *Clostridium difficile* | ATCC 1382 | 37 | Aerobic | BHI | - |
| *Enterococcus faecium* | DPC 4898 | 37 | Anaerobic | MRS | - |
| *Enterococcus mundtii* | LMG 10748 | 37 | Anaerobic | MRS | - |
| *Lactobacillus delbrueckii subsp. Bulgaricus* | LMG 6901 | 37 | Anaerobic | MRS | ++ |
| *Lactobacillus farraginis* | DSM 18382 | 30 | Anaerobic | MRS | ++ |
| *Lactobacillus fermentum* | APC 2582 | 37 | Anaerobic | MRS | + |
| *Lactobacillus nodensis* | DSM 19682 | 30 | Anaerobic | MRS | + |
| *Lactobacillus plantarum* | DSM 13278 | 37 | Anaerobic | MRS | - |
| *Lactococcus lactis* | APC 706 | 30 | Aerobic | GM17 | - |
| *Lactococcus lactis* | DPC 3147 | 30 | Aerobic | GM17 | - |
| *Listeria monocytogenes* | 1416 | 37 | Aerobic | BHI | + |
| *Listeria monocytogenes* | DPC 6893 | 37 | Aerobic | BHI | |
| *MRSA* | DPC 5645 | 37 | Aerobic | BHI | - |
| *Pseudomonas aeruginosa* | APC 2064 | 37 | Aerobic | BHI | - |
| *Salmonella typhimurium* | LT2 | 37 | Aerobic | BHI | - |
| *Staphylococcus aureus* | C55 | 37 | Aerobic | BHI | - |
| *Staphylococcus aureus* | R963 | 37 | Aerobic | BHI | - |
| *Staphylococcus epidermidis* | DPC 5990 | 37 | Aerobic | BHI | - |
| *Staphylococcus simulans* | APC 3482 | 37 | Aerobic | BHI | - |
| *Streptococcus agalactiae* | APC 1055 | 37 | Aerobic | BHI | ++ |
| *Streptococcus agalactiae* | ATCC 13813 | 37 | Aerobic | BHI | ++ |
| *Streptococcus bovis* | DPC 6491 | 37 | Aerobic | BHI | ++ |
| *Streptococcus dysgalactiae* | APC 3484 | 37 | Aerobic | BHI | ++ |
| *Streptococcus hyointestinalis* | DPC 6484 | 37 | Anaerobic | GM17 | - |
| *Streptococcus uberis* | DPC 5344 | 37 | Aerobic | BHI | - |

### Purified antimicrobial peptides

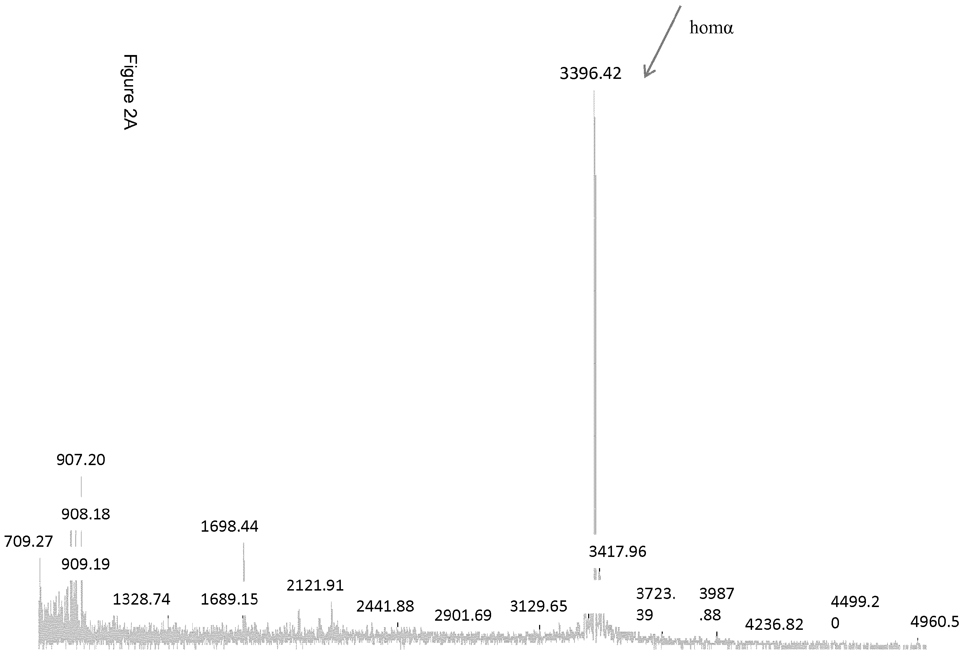

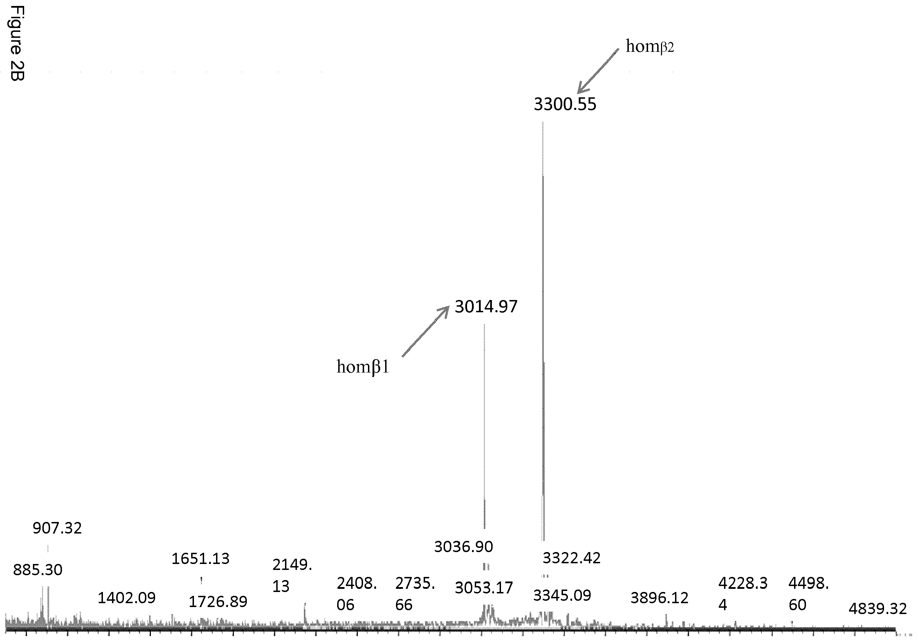

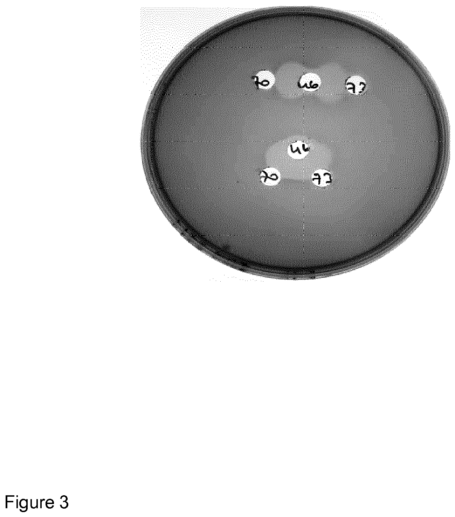

Two active fractions were obtained after purification by RP-HPLC analysis. MALDI-TOF MS identified a molecular mass of 3396.4Da in the first fraction. However, in the second fraction two molecular masses of 3300.5Da and 3014.9Da were identified. It was not immediately apparent which masses were responsible for the bacteriocin activity in this second partially purified fraction as it was not possible to separate the two peptides of similar mass. However, three core peptides of interest were identified within the bacteriocin operon after genome sequencing of S. hominis APC 3675, APC 2924 and APC 2925. Subsequently these three peptides were suggested to be responsible for the antimicrobial activity displayed against S. agalactiae APC1055 and Lb. delbrueckii subsp. bulgaricus LMG 6901 (Figures 2 and 3).

### MIC results and peptide stability

Results from the MIC assay performed against S. agalactiae APC 1055 revealed that homicin α and β peptides, at a 1:1 ratio, had a MIC cut off value of 6.25 µM. In terms of stability, homicin retained antimicrobial activity after treatment at 100 °C for 15 min. The bacteriocin was also found to be susceptible to digestion by α-chymotrypsin and proteinase K, indicating the proteinaceous nature of the bacteriocin.

### Identification of plasmid encoded, two-component lantibiotic operon

Interestingly, after analysis of the putative lantibiotic operon, three putative lantibiotic encoding structural genes were identified; one homA1 structural α peptide and two homA2 structural β-peptides (Table 2). This contig containing the bacteriocin operon spans approximately 43 kb.

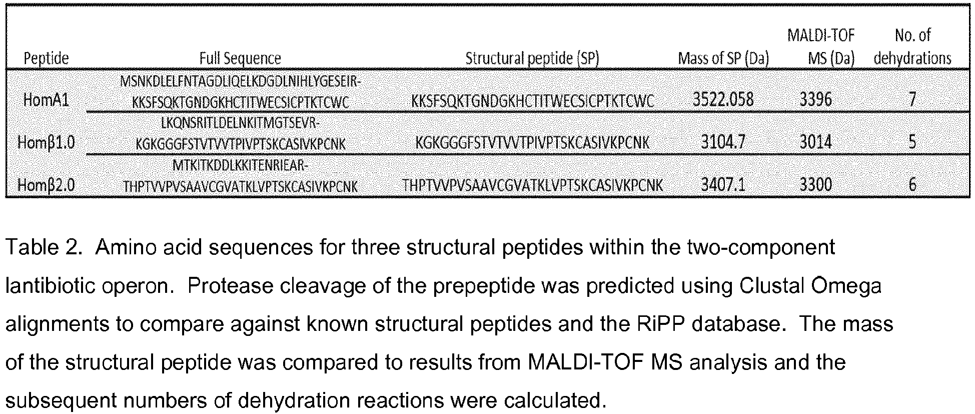

Due to the extent of nucleotide coverage and the presence of plasmid encoding elements, the lantibiotic operon is thought to be plasmid encoded. The following genetic elements suggest that the novel lantibiotic is plasmid encoded: 1) at the beginning of the DNA sequence a replication initiation protein (repA) was detected with 94% identity to S. hominis repA, 2) a plasmid mobilization relaxosome protein (MobC) was detected with 80% identity to MobC, 3) a plasmid recombination protein (PRP) was detected with 99% identity to many PRPs in Staphylococcus spp. and 4) a replication initiation protein (repA) was detected encoding a putative DNA relaxase NicK with 99% identity to NicK found in Staphylococcus aureus.

Within the bacteriocin encoding segment of the plasmid, a two-component lantibiotic was identified. A 66-amino-acid putative α-peptide was first identified, followed by two similar 54-amino-acid β-peptides, homβ1 and homβ2. These predicted structural β-peptides were found to have 54.8% identity to one another. Initial Bagel4 output of the two-component lantibiotic found the unmodified homicin α peptide be 40% similar to haloduracin α, and the unmodified homicin β peptides to be 30% similar to haloduracin β. However, after prediction of the protease cleavage site in homα (Arg-Lys), the core structural α-peptide was aligned against previously identified α-peptides and the following result was obtained by Clustal Omega-2.1: 40% identity to smbA1, and 38.5% identity to ItnA1, sacA1 and bhtA1 (Figure 4). Similarly, the two residues resembling the core β-peptides were aligned against those previously identified two-component β-peptides. Homβ1 was found to display 30.7% identity to lichenicidenA2, and homβ2 was found to display 34.8% identity to haloduracin β (Figure 4).

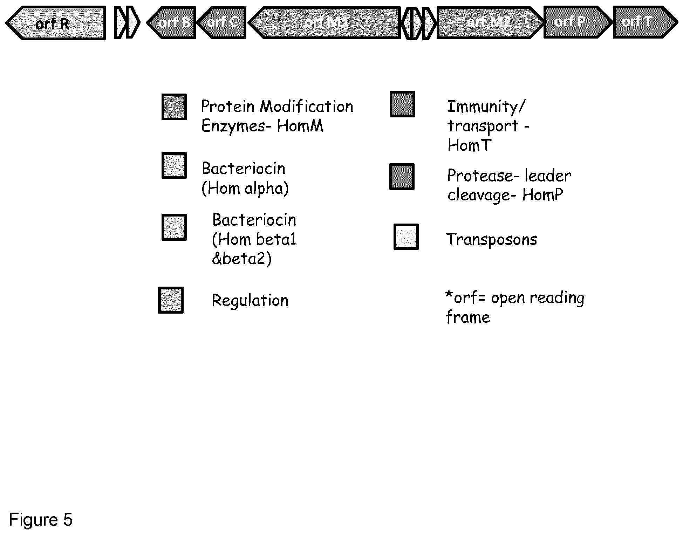

Furthermore, Bagel4 identified two lantibiotic modification enzymes in the homicin operon (Figure 5). The order of the genes in the operon would suggest that ORF M1 (homM1) is the modification enzyme associated with homα, and ORF M2 (homM2) is the modification enzyme associated with homβ1 and homβ2. Amino acid alignment of homM1 and homM2 displayed only 25% identity to one another. The amino acid sequences of homM1 and homM2 were subsequently input to the Blastp suite where the sequences were found to display 38% identity to a LanM modification enzyme in Virgibacillus dokdensis DSW-10, a novel strain isolated from sea water at Dokdo, an island in the East Sea, Korea (Yoon et al., 2005).

In terms of lanthipeptide immunity, ORF B, ORF C and ORF T were found to encode ABC transporter-related peptides which are likely to be involved in bacteriocin transport and immunity (Figure 5). ORF B encoded an ABC-2 membrane transporter family (pfam01061), while ORF C displayed 52% identity to the ABC transporter ATP-binding protein in Staphylococcus delphini 8086. This ABC transporter was also found to be part of an ATP-binding cassette domain of the bacitracin-resistance transporter (BcrA), representing ABC transporters involved in peptide antibiotic resistance. ORF T was found to encode an ABC-type multidrug transport system, which displayed 50-53% identity to previously identified ABC transporter ATP-binding proteins within the Staphylococcus species in NCBI (specifically Staphylococcus saprophyticus). Interestingly, located just downstream of the transporter homT, a DnaB domain-containing protein responsible for replication initiation and membrane attachment was identified. ORF R displayed 90% identity to Staph. equorum with a specific hit on BLAST NCBI to polymerase and histidinol phosphatase domain of histidinol phosphate phosphatase. This enzyme plays a role in histidine metabolism; specifically catalysing the penultimate step of the histidine biosynthesis pathway: i.e. the dephosphorylation of histidinol phosphate to histidinol, the direct precursor of histidine (Le Coq, Fillinger and Aymerich, 1999), suggesting it to be a putative regulatory protein for the homicin operon. NCBI BLAST results also revealed ORF R encoded a putative AbiEii toxin, type IV toxin/antitoxin system, responsible for an abortive phage resistance system (pfam: PF08843) (Dy et al., 2014).

The lantibiotic protease (homP), which cleaves the structural peptides within the lantibiotic operon, was located downstream of the homβ structural peptides (ORF P), which was further analysed to reveal a peptidase S8 family domain. This lantibiotic specific protease is known to cleave the N-terminal leader peptides from lantiobiotics. Further analysis of the peptidase domain revealed a putative subtilisin-like, serine protease (AprE) conserved region encoded within the homP genetic machinery (see pfam00082). The blastp suite found homP to have 40% identity to other Bacillus cereus peptidases on the NCBI database. A gene responsible for the transport of the novel antimicrobial was also identified.

### Additional In silico results from draft genome S. hominis APC 3675

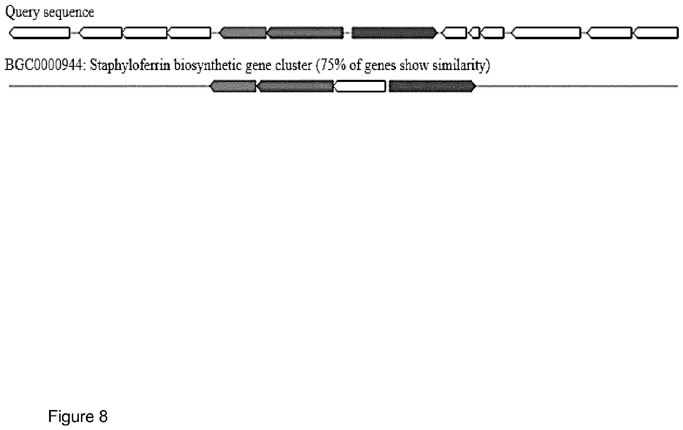

In addition to a novel lantibiotic operon, a biosynthetic gene cluster was identified with 75% similarity to a prevalent siderophore, staphyloferrin A (Cotton et al., 2009), using antiSMASH (see Figure 8). Thus, in response to iron limitation the presence of a potential siderophore would benefit the survival of S. hominis strains APC 3675, APC 2924 and APC 2925. No virulence genes were found using VirulenceFinder-1.5. However, ResFinder-3.0 found genes responsible for beta-lactam resistance (blaZ-95.9% identity), macrolide resistance (mph- 99.9% identity), streptogramin A resistance (msr-99.0% identity) and tetracycline resistance (tet-99.9% identity). Moreover, PlasmidFinder-1.3 was able to detect a RepC cassette with 100% identity to a staphylococcal shuttle vector. This potential plasmid was 4566bp in length and contained the TetK gene responsible for tetracycline resistance, with 99% similarity to the tetracycline resistance efflux pumps found using the Basic Local Alignment Search Tool (BLAST).

### Homology between bacteriocins

Comparisons of the structural peptides associated with homicin were performed using Clustal Omega-2.1. Previously identified two-component bacteriocins were used to align the structural peptides associated with homicin (Figure 4). Results indicate that homicin displays a similar degree of homology displayed amongst other bacteriocins. The homology in lanthionine and methyl-lanthionine bridge formation suggests a structural similarity in each of the peptides. Interestingly, the regions of conservation observed at the C-terminus of the β peptides suggest a conserved pattern of lanthionine and methyl-lanthionine bridge formation. However, a much lower degree of conservation was found at the N-terminus of the bacteriocins.

### Bacteriocin structure prediction and analysis

MALDI-TOF MS analysis of active HPLC fractions led to the identification of molecular masses 3396, 3014 and 3300.5 Da of predicted α, β1 and β2 structural peptides, respectively (Figure 2). The predicted mass of the α-peptide, based on the amino acid sequence from the draft genome, was 3522.10 Da (Table 2); the difference between the predicted and observed 3396 Da mass correlates with the loss of seven water residues associated with the formation of lanthionine and methyllanthionine bridges and the dehydration of Ser-9.

To predict the α-peptide structure, Ltnα was used as a template. Theoretically the formation of a lanthionine bridge may occur between Ser-11 and Cys-21, whilst methyllanthionine bridges could form between Thr-22 and Cys-27, Thr-24 and Cys-30, Thr-32 and Cys-35, as well as between Thr-34 and Cys-37, whilst Ser-9 is dehydrated to its respective Dha residue and Ser-28 remains unaltered (Figures 4 and 6).

The first β peptide, located downstream to the alpha peptide in the bacteriocin operon, had a predicted mass of 3104.7 Da (homβ1). The predicted mass had a difference of 90 Da from the mass detected by MALDI TOF MS (Table 2). This mass difference corresponds with the prediction of five dehydrations in the peptide. Using the structures of halβ and lichβ peptides as templates, the peptide was predicted to form bridges between Thr-26 and Cys-29, and Ser-31 and Cys-36. This would result in Ser-15, Thr-16, and Thr-21 being dehydrated to their respective Dha and Dhb residues, whilst Thr-18 and Ser-27 remain unaltered (Figures 4 and 6).

Finally, the second β peptide located in the bacteriocin operon (homβ2) had a predicted mass of 3407.1 Da, a difference of 107 Da from the 3300 Da mass detected by MALDI-TOF MS (Table 2, Figure 4). This mass difference corresponds with the prediction of six dehydrations in the peptide. The peptide was predicted to form bridges between Ser-13 and Cys-17, Thr-26 and Cys-29, and Ser-31 and Cys-36. This would result in Thr-4, Thr-7 and Thr-21 being dehydrated to their respective Dha and Dhb residues, whilst Ser-27 would remain unaltered (Figures 4 and 6).

### Comparison of three S. hominis strains

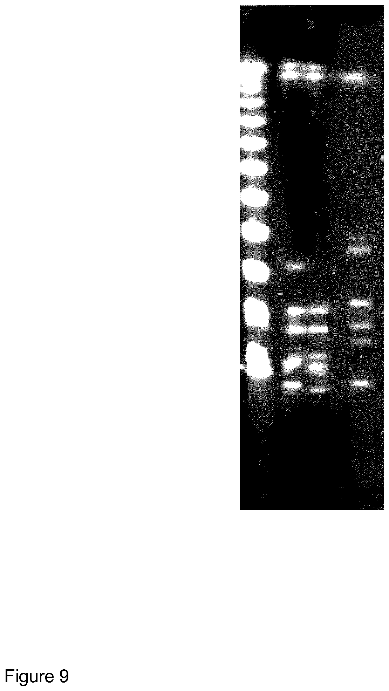

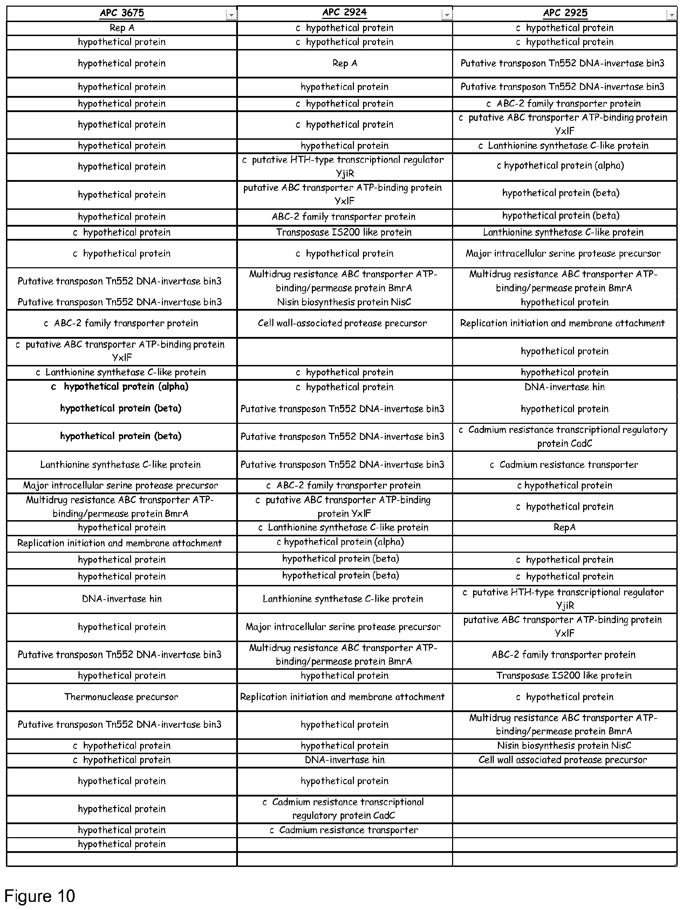

As the bacteriocin operons of APC 3675, APC 2924 and APC 2925 were identical, the next step was to investigate if there were differences between the three strains. Three approaches were taken - firstly PFGE procedure was carried out to view genetic fingerprint of the strains which revealed differences in pulsotypes of the strains (Figure 9). Secondly, a comparative table of genes present on the homicin contig was created (Figure 10). This table highlights what genes were present in one strain that were not present in another. Thirdly, colony MS was carried out individually on all three cultures. An extra peak at ∼2370Da was observed in APC 2924 and APC 2925, but this mass was not present in APC 3675. From these three comparative methods, differences were found between all three strains. However, APC 3675 appears to be the most unique of the three S. hominis strains in this study.

### Discussion

Homicin represents a novel, plasmid encoded, narrow spectrum lantibiotic. Two different skin associated microbiomes (vagina and toe web-space), present in unrelated individuals from two different cohorts, highlights the dominance of this bacteriocin in the human population. Genome sequencing of the three S. hominis strains: APC 3675, APC 2924 and APC 2925 led to the identification of the genetic elements required for plasmid replication, conjugal transfer, lantibiotic production and immunity. The plasmid was found to encode several Tn552 transposons, flanking either end of the bacteriocin encoding segment of the plasmid DNA. Tn552, also described as a transposon DNA-invertase, was found to display 94-95% identity to other transposon elements in S. epidermidis and S. aureus strains in NCBI. The transposon located downstream of the bacteriocin operon was further classified as a site-specific DNA recombinase related to DNA invertase Pin (PinE), which allows for DNA recombination in inverted repeat (Kazuhiro et al., 1985). Moreover, Tn552 encodes a helix-turn-helix domain of Hin. The Hin recombinase induces the site-specific inversion of a DNA segment encoding a promoter, which controls the alternate expression of two genes by reversibly switching orientation (Mack et al., 1990). This interesting feature of the homicin operon would explain the directionality of the LanM modification enzymes as interpreted by Bagel4 (Figure 5) and Artemis. Furthermore, the MobC plasmid relaxosome protein displayed 90% identity to many Staphylococcus epidermidis strains found in the NCBI database, suggesting that conjugative transfer of the mobile plasmid may have originated from an S. epidermidis skin isolate. It is not surprising that S. hominis and S. epidermidis would be involved in the conjugative transfer of plasmids as both organisms are commensal habitants of the skin microbiome. To date, there have been many two-component lantibiotics found to be encoded on plasmids. For example, staphylococcin C55 locus is located on a 32 kb plasmid in the strain S. aureus C55 (Navaratna et al., 1998), but has also been identified on a 38 kb pETB plasmid in S. aureus TY4 (Yamaguchi et al., 2001). Lacticin 3147 has also been identified on a 60.2 kb plasmid (Dougherty et al., 1998). Analysis of the structural peptides responsible for antimicrobial production resulted in the identification of three putative peptides. The first homα-peptide was easily identifiable due to the degree of homology and conserved regions similar to other known two-component lantibiotics. However, the identification of two potential β-peptides was noteworthy and may be explained by the conjugative transfer of mobile genetic elements on the plasmid. It is predicted that homα binds to lipid II, thereby inhibiting cell wall synthesis through the prevention of transglycosylation. Subsequently, pore formation occurs with a rapid efflux of K+ and phosphate ions due to the presence of either homβ peptides. Similar to the findings described in the homicin encoded operon, the identification of a third antimicrobial peptide occurred during the purification of staphylococcin C55 (Navaratna et al., 1998). Navaratna et al. described the third, γ-peptide, after inhibitory activity was detected in three distinct regions of eluent fluid after HPLC fractionation. The three peptides (staphylococcins C55α, C55β, and C55γ) were found to be responsible for the inhibitory activity of S. aureus C55. However, there was no evidence of antibacterial interaction or synergy between staphylococcin C55γ with either C55α or C55β (Navaratna et al., 1998).

A more recent study identified a novel two-component lantibiotic termed thusin, that consisted of Thsα, Thsβ, and Thsβ' (mutation of Thsβ), isolated from a B. thuringiensis strain BGSC 4BT1 (Xin et al., 2016). The sequences of ThsA2 (62 aa) and ThsA2' (61 aa) were nearly identical (95% identity) and Xin et al. found that the mature peptides (Thsα and Thsβ (or Thsβ')) acted synergistically to inhibit several Gram-positive pathogens; with maximized bioactivity at a 1:1 ratio. Thus, further investigation and purification of the homβ lyophilised fraction will provide information on the synergistic effect between each homβ-peptides, in combination with the identified homα-peptide.

Indeed, the predicted structure in both α and β peptides display a high degree of similarity to other two-component lantibiotics. For example, the first three cysteine residues in homa are conserved among other two-component lantibiotics, with the exception of two additional cysteine residues at Cys-35 and Cys-37 (Figure 5). Similarly, the cysteine residue at Cys-29 in the two β-peptides appears to be conserved among haloduracin, licheniciden, lacticin 3147 and plantaricin W β-peptides; however, the last cysteine residue at Cys-36 appears to form a lanthionine bridge with four amino acids; which is unique to the homicin lanthipeptide. When both β peptides are compared to one another, it is interesting to note the additional lanthionine bridge formed in homβ2 (Figures 5 and 6). This lanthionine bridge is formed between Ser-13 and Cys-17, which appears to be conserved with the Ser-20 and Cys-24 bridge formation in other two-component bacteriocins (Figure 4). Subsequently, due to this additional conserved region in homβ2, it would suggest that this β peptide may prove to display a higher degree of synergy with the homicin α peptide.

The surprising similarity of the LanM modification enzyme in V. dokdensis DW-10 to the homM modification enzymes lead to further investigation of the draft genome assembly for this strain. After downloading the nucleotide FASTA sequence, accessible from the NCBI database, for V. dokdensis DW-10, Bagel3 was used to search for a putative bacteriocin operon. Presumptive identification of a lantibiotic operon was found with 42% and 38% identity to haloduracin and lichenicidenVK21 α-peptides (see Figure 12). However, to date there have been no lantibiotics published in the literature for V. dokdensis DW-10, suggesting that this method of screening for LanM similarities has led to the identification of a putative lantibiotic operon.

When comparing the three S. hominis strains by PFGE to identify genetic differences, all three strains had different pulsotypes. APC 3675 (vaginal isolate) had the most different pulsotype of the three, while APC 2925 (toe web-space skin isolate) had one extra Sma1 DNA band when compared to APC 2924 (toe web-space skin isolate from a different subject), demonstrating that the bacteriocin operon is conserved between three genetically distinct isolates. The fact that homicin was produced in three different strains of S. hominis, in different locations of the body, suggests a certain dominance associated with this particular bacteriocin. As the homicin operon has been verified to be on a plasmid, the inventors hypothesise that this gene cassette may be transferred via bacterial conjugation or horizontal gene transfer.

### References

Altena K, Guder A, Cramer C and Bierbaum G. 2000. Biosynthesis of the lantibiotic mersacidin: organization of a type B lantibiotic gene cluster. Appl Environ Microbiol. 66(6):2565-71. doi: 10.1128/AEM.66.6.2565-2571.2000.
Arnison PG, Bibb MJ, Bierbaum G, Bowers AA, Bugni TS, Bulaj G, et al. 2012. Ribosomally synthesized and posttranslationally modified peptide natural products: overview and recommendations for a universal nomenclature. Nat Prod Rep. doi:10.1039/C2NP20085F.
Azad MB, Konya T, Persaud RR, Guttman DS, Chari RS, Field CJ et al. 2016. Impact of maternal intrapartum antibiotics, method of birth and breastfeeding on gut microbiota during the first year of life: a prospective cohort study. BJOG. 123(6):983-93. doi: 10.1111/1471-0528.13601.
Begley M, Cotter PD, Hill C, Ross RP. 2009. Identification of a Novel two-peptide lantibiotic, lichenicidin, following rational genome mining for LanM proteins . Appl Environ Microbiol. 75(17):5451-5460. doi:10.1128/AEM.00730-09.
Ben Zakour NL, Beatson SA, van den Broek AHM, Thoday KL, Fitzgerald JR. 2012. Comparative Genomics of the Staphylococcus intermedius Group of Animal Pathogens. Frontiers in Cellular and Infection Microbiology. 2:44. doi:10.3389/fcimb.2012.00044.
Berendonk TU, Manaia CM, Merlin C, Fatta-Kassinos D, Cytryn E, Walsh F, et al. 2015. Tackling antibiotic resistance: the environmental framework. Nat Rev Microbiol. p13(5):310-7. doi: 10.1038/nrmicro3439.
Bierbaum G, Götz F, Peschel A, Kupke T, van de Kamp M and Sahl HG. 1996. The biosynthesis of the lantibiotics epidermin, gallidermin, Pep5 and epilancin K7. Antonie Van Leeuwenhoek. 69(2):119-127.
Borghesi A, Stronati M, Fellay J. 2017. Neonatal Group B Streptococcal Disease in Otherwise Healthy Infants: Failure of Specific Neonatal Immune Responses. Frontiers in Immunology. 8:215. doi:10.3389/fimmu.2017.00215.
Carattoli A, Zankari E, Garcia-Fernandez A, Voldby Larsen M, Lund O, et al. 2014. PlasmidFinder and pMLST: in silico detection and typing of plasmids. Antimicrob. Agents Chemother. 58(7):3895-903. doi: 10.1128/AAC.02412-14.
Chiller K, Selkin BA, Murakawa GJ. 2001. Skin microflora and bacterial infections of the skin. Journal of Investigative Dermatology Symposium Proceedings. 6, 3: 170-174.
Collins FW, O'Connor PM, O'Sullivan O, Rea MC, Hill C, Ross RP. 2016. Formicin - a novel broad-spectrum two-component lantibiotic produced by Bacillus paralicheniformis APC 1576. Microbiology. 162(9):1662-1671. doi: 10.1099/mic.0.000340.
Cotter PD, Ross RP, Hill C. 2013. Bacteriocins - a viable alternative to antibiotics? Nat Rev Microbiol. p11(2):95-105. doi: 10.1038/nrmicro2937.
Cotton JL, Tao J, Balibar CJ. 2009. Identification and characterization of the Staphylococcus aureus gene cluster coding for staphyloferrin A. Biochemistry. 48(5):1025-35. doi: 10.1021/bi801844c.
Danesh A, Ljungh Å, Mattiasson B, Mamo G. 2017. Synergistic effect of haloduracin and chloramphenicol against clinically important Gram-positive bacteria. Biotech Rep. 13:37-41. doi:10.1016/j.btre.2016.12.008.
Dougherty BA, Hill C, Weidman JF, Richardson DR, Venter JC, Ross RP. 1998. Sequence and analysis of the 60 kb conjugative, bacteriocin-producing plasmid pMRC01 from Lactococcus lactis DPC3147. Mol Microbiol. 29(4):1029-38. PubMed PMID: 9767571.
Dy RL, Przybilski R, Semeijn K, Salmond GP, Fineran PC. 2014. A widespread bacteriophage abortive infection system functions through a Type IV toxin-antitoxin mechanism. Nucleic Acids Res. 42(7):4590-605. doi: 10.1093/nar/gkt1419.
Grice, E. A. and Segre, J. A. (2011) 'The skin microbiome', Nature Reviews Microbiology, 9(4), pp. 244-253. doi: 10.1038/nrmicro2537.
Fernández-López C, Bravo A, Ruiz-Cruz S, Solano-Collado V, Garsin DA, Lorenzo-Diaz F et al. 2015. Mobilizable Rolling-Circle Replicating Plasmids from Gram-Positive Bacteria: A Low-Cost Conjugative Transfer. Plasmids: Biology and Impact in Biotechnology and Discovery. ch15, p 257-276. doi:10.1128/microbiolspec.PLAS-0008-2013.
Field D, Connor PM, Cotter PD, Hill C, Ross RP. 2008. The generation of nisin variants with enhanced activity against specific gram-positive pathogens. Mol Microbiol. 69(1):218-30. doi: 10.1111/j.1365-2958.2008.06279.x.
Field D, Cotter PD, Hill C, Ross RP. Bioengineering lantibiotics for therapeutic success. frontiers in microbiology. 2015;6:1363. doi:10.3389/fmicb.2015.01363.
Garg N, Tang W, Goto Y, Nair SK, van der Donk WA. 2012. Lantibiotics from Geobacillus thermodenitrificans. Proceedings of the National Academy of Sciences of the United States of America. 109(14):5241-5246. doi:10.1073/pnas.1116815109.
Gopal Rao G, Nartey G, McAree T, O'Reilly A, Hiles S, Lee T, et al. 2017. Outcome of a screening programme for the prevention of neonatal invasive early-onset group B Streptococcus infection in a UK maternity unit: an observational study. BMJ Open, 7(4), e014634. http://doi.org/10.1136/bmjopen-2016-014634.
Götz F, Perconti S, Popella P, Werner R, Schlag M. 2014. Epidermin and gallidermin: Staphylococcal lantibiotics. Int J Med Microbiol. 304(1):63-71. doi: 10.1016/j.ijmm.2013.08.012.
Holo H, Jeknic Z, Daeschel M, Stevanovic S and Nes IF. 2001. Plantaricin W from Lactobacillus plantarum belongs to a new family of two-peptide lantibiotics. Microbiology. 147(Pt 3):643-51.
Holo H, Jeknic Z, DaeschelM, Stevanovic S, Nes IF. 2001. Plantaricin W from Lactobacillus plantarum belongs to a new family of two-peptide lantibiotics. Microbiology. 147(Pt 3):643-51. Il Kim, P. et al. (2010) 'Biochemical and Biophysical Research Communications Characterization and structure identification of an antimicrobial peptide , hominicin , produced by Staphylococcus hominis MBBL 2 - 9', Biochemical and Biophysical Research Communications. Elsevier Inc., 399(2), pp. 133-138. doi: 10.1016/j.bbrc.2010.07.024.
Joensen KG, Scheutz F, Lund O, Hasman H, Kaas RS, Nielsen EM,et al. 2014. Real-time whole-genome sequencing for routine typing, surveillance, and outbreak detection of verotoxigenic Escherichia coli. J Clin Micobiol. 52(5): 1501-1510.
Kazuhiro K. and Toshifumi N. 1985. A gene for DNA invertase and an invertible DNA in Escherichia coli K-12. Gene 34(2), pp.343-350.
Kleinheinz KA, Joensen KG, Larsen MV. 2014. Applying the ResFinder and VirulenceFinder web-services for easy identification of acquired antibiotic resistance and E. coli virulence genes in bacteriophage and prophage nucleotide sequences. Bacteriophage. 4:e27943. doi:10.4161/bact.27943.
Larsen MV, Cosentino S, Rasmussen S, Friis C, Hasman H, Marvig RL, et al. 2012. Multilocus sequence typing of total-genome-sequenced bacteria. J Clin Microbiol. 50(4):1355-61. doi: 10.1128/JCM.06094-11.
Mack DP, Sluka JP, Shin JA, Griffin JH, Simon MI, Dervan PB. 1990. Orientation of the putative recognition helix in the DNA-binding domain of Hin recombinase complexed with the hix site. Biochemistry 29(28):6561-7.
Mathur H, Field D, Rea MC, Cotter PD, Hill C, Ross RP. Bacteriocin-antimicrobial synergy: a medical and food perspective. Frontiers in Microbiology. 2017;8:1205. doi:10.3389/fmicb.2017.01205.
McAuliffe O, Hill C, Ross RP. 2000. Each peptide of the two-component lantibiotic lacticin 3147 requires a separate modification enzyme for activity. Microbiology. 146:2147-54. doi: 10.1099/00221287-146-9-2147.
McAuliffe O, Ross RP, Hill C. Lantibiotics: structure, biosynthesis and mode of action. FEMS Microbiol Rev. 2001 May;25(3):285-308.
McClerren AL, Cooper LE, Quan C, Thomas PM, Kelleher NL and van der Donk WA. 2006. Discovery and in vitro biosynthesis of haloduracin, a two-component lantibiotic. Proc Natl Acad Sci U S A. 103(46):17243-8.
Navaratna MA, Sahl HG and Tagg JR. 1998. Two-component anti-Staphylococcus aureus lantibiotic activity produced by Staphylococcus aureus C55. Appl Environ Microbiol. 64(12):4803-8
Navaratna MADB, Sahl H-G, Tagg JR. 1998. Two-component anti-Staphylococcus aureus lantibiotic activity produced by Staphylococcus aureus C55. Applied and Environmental Microbiology. 64(12):4803-4808.
O'Connor EB, Cotter PD, O'Connor P, O'Sullivan O, Tagg JR, Ross RP, et al. 2007. Relatedness between the two-component lantibiotics lacticin 3147 and staphylococcin C55 based on structure, genetics and biological activity. BMC Microbiol. 2;7:24.
Ohlsson A, Shah VS. 2009. Intrapartum antibiotics for known maternal group B streptococcal colonization. Cochrane Database Syst Rev. (3):CD007467. doi: 10.1002/14651858.CD007467.pub2.
Otto, M. (2010) 'Staphylococcus colonisation of the skin and antimicrobial peptides', Expert Rev Dermatol., 5(2), pp. 183-195. doi: 10.1586/edm.10.6.Staphylococcus.
.Patras KA, Wescombe PA, Rösler B, Hale JD, Tagg JR, Doran KS. 2015. Streptococcus salivarius K12 limits group b streptococcus vaginal colonization. Camilli A, ed. Infection and Immunity. 83(9):3438-3444. doi:10.1128/IAI.00409-15.
Pieterse R, Todorov SD, Dicks LM. 2008. Bacteriocin ST91KM, produced by Streptococcus gallolyticus subsp. macedonicus ST91KM, is a narrow-spectrum peptide active against bacteria associated with mastitis in dairy cattle. Can J Microbiol. 54(7):525-31. doi: 10.1139/w08-040.
Poyart C, Tazi A, Reglier-Poupet H, et al. Multiplex PCR Assay for Rapid and Accurate Capsular Typing of Group B Streptococci. Journal of Clinical Microbiology. 2007;45(6):1985-1988. doi:10.1128/JCM.00159-07.
Ramana KV, Vikram G, Wali PP, Anand K, Rao SD, Mani RMS, Sarada VCH and Rao R. 2014. Non diphtheritic corynebacteria (NDC) and their clinical significance: clinical microbiologist's perspective. Am J Epidemiol. 2014;2:83-7.
Robins E, Haile-Selassie T. 2001. Corynebacterium xerosis sepsis in a pediatric patient with sickle cell disease (a case report). Clinical pediatrics. 40(3):181.
Ryan MP, Rea MC, Hill C and Ross RP. 1996. An application in cheddar cheese manufacture for a strain of Lactococcus lactis producing a novel broad-spectrum bacteriocin, lacticin 3147. Appl Environ Microbiol. 62:612-619.
Sasikumari O and Thomas, S. 2018. Isolation of Corynebacterium xerosis from clinical specimens: A case series. Journal of The Academy of Clinical Microbiologists. 20(1), p.43.
Sawa N, Wilaipun P, Kinoshita S, et al. Isolation and characterization of enterocin w, a novel two-peptide lantibiotic produced by Enterococcus faecalis NKR-4-1. Applied and Environmental Microbiology. 2012;78(3):900-903. doi:10.1128/AEM.06497-11.
Siegers K, Heinzmann S, and Entian KD. 1996. Biosynthesis of Lantibiotic Nisin: posttranslational modification of its prepeptide occurs at a multimeric membrane-associated lanthionine synthetase complex. J Biol Chem. 271: 12294. doi:10.1074/jbc.271.21.12294.
Sievers F, Wilm A, Dineen D, et al. 2011. Fast, scalable generation of high-quality protein multiple sequence alignments using Clustal Omega. Molecular Systems Biology. 7:539. doi:10.1038/msb.2011.75.
Singh M, Sareen D. Novel LanT Associated Lantibiotic Clusters Identified by Genome Database Mining. Biswas I, ed. PLoS ONE. 2014;9(3):e91352. doi:10.1371/journal.pone.0091352.
Sung, C. et al. (2010) 'Probiotic potential of Staphylococcus hominis MBBL 2 - 9 as anti-Staphylococcus aureus agent isolated from the vaginal microbiota of a healthy woman', 108, pp. 908-916. doi: 10.1111/j.1365-2672.2009.04485.x.
Van Heel AJ, de Jong A, Song C, Viel JH, Kok J, Kuipers OP. 2018. BAGEL4: a user-friendly web server to thoroughly mine RiPPs and bacteriocins, Nucleic Acids Research, Volume 46, Issue W1, 2 July 2018, Pages W278-W281, https://doi.org/10.1093/nar/gky383.
Verani JR, McGee L, Schrag SJ; Centers for Disease Control and Prevention (CDC). 2010. Prevention of perinatal group B streptococcal disease--revised guidelines from CDC. Morb Mortal Wkly Rep. 59(RR-10):1-36.
Waterhouse AM, Procter JB, Martin DMA,Clamp M, Barton GJ. 2009. Jalview Version 2 - a multiple sequence alignment editor and analysis workbench. Bioinformatics. 25(9): 1189-91 doi: 10.1093/bioinformatics/btp033.
Weber T, Blin K, Duddela S, et al. 2015. antiSMASH 3.0-a comprehensive resource for the genome mining of biosynthetic gene clusters. Nucleic Acids Research. 43(Web Server issue):W237-W243. doi:10.1093/nar/gkv437.
World Health Organization (WHO). 2014. Antimicrobial resistance: global report on surveillance. p257; ISBN 978 92 4 156474 8.
Xin B, Zheng J, Liu H, Li J, Ruan L, Peng D, et al. 2016. Thusin, a Novel Two-Component Lantibiotic with Potent Antimicrobial Activity against Several Gram-Positive Pathogens. Front Microbiol. 7:1115. doi: 10.3389/fmicb.2016.01115.
Yang K, Kruse RL, Lin WV, Musher DM. 2018. Corynebacteria as a cause of pulmonary infection: a case series and literature review. Pneumonia. 10(1):10.
Yamaguchi T, Hayashi T, Takami H, Ohnishi M, Murata T, Nakayama K. 2001. Complete nucleotide sequence of a Staphylococcus aureus exfoliative toxin B plasmid and identification of a novel ADP-ribosyltransferase, EDIN-C. Infect Immun. 69(12):7760-71.
Yoon JH, Kang SJ, Lee SY, Lee MH, Oh TK. 2005. Virgibacillus dokdonensis sp. nov., isolated from a Korean island, Dokdo, located at the edge of the East Sea in Korea. Int J Syst Evol Microbiol. 55(Pt 5):1833-7.
Zankari E, Hasman H, Cosentino S, Vestergaard M, Rasmussen S, Lund O, Aarestrup FM, Larsen MV. 2012. Identification of acquired antimicrobial resistance genes. J Antimicrob Chemother. doi: 10.1093/jac/dks261.
Zasada AA and Mosiej E. 2018. Contemporary microbiology and identification of Corynebacteria spp. causing infections in human. Letters in applied microbiology. 66(6):472-83.

## Claims

1. An isolated lantibiotic comprising:
- amino acid sequence SEQ. ID No. 2 or a variant amino acid sequence having at least 90% amino acid sequence identity thereto, and
- at least one of:
- amino acid sequence SEQ. ID No. 4 or a variant amino acid sequence having at least 90% amino acid sequence identity thereto, and
- amino acid sequence SEQ. ID. No 6 or a variant amino acid sequence having at least 90% amino acid sequence identity thereto.

2. The lantibiotic as claimed in claim 1, wherein the lantibiotic comprises both:
- amino acid sequence SEQ. ID No. 4 or a variant amino acid sequence having at least 90% amino acid sequence identity thereto, and
- amino acid sequence SEQ. ID. No 6 or a variant amino acid sequence having at least 90% amino acid sequence identity thereto.

3. The lantibiotic as claimed in claim 1 or 2, wherein the lantibiotic comprises:
- amino acid sequence SEQ. ID No. 2,
- amino acid sequence SEQ. ID No. 4, and
- amino acid sequence SEQ. ID. No 6.

4. The lantibiotic as claimed in any one of claims 1 to 3 for use in the treatment or prevention of infection by Group B Streptococcus.

5. The lantibiotic as claimed in any one of claims 1 to 3 for use in the treatment or prevention of infection by Corynebacterium xerosis.

6. An isolated S. hominis strain selected from the group consisting of S. hominis APC 3675 deposited under NCIMB 43358, S. hominis APC 2924 deposited under NCIMB 43356 and S. hominis APC 2925 deposited under NCIMB 43357.

7. An isolated amino acid sequence comprising amino acid sequence SEQ. ID. No. 2, 4 or 6, or a variant amino acid sequence having at least 90% amino acid sequence identity thereto.

8. A method for producing the lantibiotic as claimed in any one of claims 1 to 3, the method comprising combining amino acid sequence SEQ. ID No. 2 or a variant amino acid sequence having at least 90% amino acid sequence identity thereto with at least one of:
- amino acid sequence SEQ. ID No. 4 or a variant amino acid sequence having at least 90% amino acid sequence identity thereto, and
- amino acid sequence SEQ. ID. No 6 or a variant amino acid sequence having at least 90% amino acid sequence identity thereto.

9. An isolated nucleotide sequence comprising SEQ ID No.15, 17 or 19 or a variant nucleotide sequence which has at least 90% sequence identity to
(a) SEQ ID No.15, 17 or 19, or
(b) a nucleotide sequence that is capable of hybridising to SEQ ID No.15, 17 or 19 under stringent conditions.

10. An isolated nucleotide sequence encoding a polypeptide comprising amino acid sequence SEQ. ID No. 2, 4 or 6.

11. A vector comprising an isolated nucleotide sequence encoding a polypeptide comprising amino acid sequences SEQ. ID No. 2, 4 and 6, optionally wherein the vector further comprises one or more of SEQ ID No. 14, 16, 18, 20, 21, 22, 23, 24, 25 and 26.

12. A host cell comprising a vector as claimed in claim 11.

13. A method of producing the lantibiotic as claimed in claim 3, the method comprising the step of cultivating at least one of the strains as claimed in claim 6 or a host cell as claimed in claim 12 under conditions suitable for production of the lantibiotic.

14. An isolated plasmid comprising SEQ ID No. 15, 17 and 19, optionally wherein the plasmid further comprises one or more of SEQ ID No. 14, 16, 18, 20, 21, 22, 23, 24, 25 and 26.

15. A biotherapeutic comprising a strain as claimed in claim 6 or a host cell as claimed in claim 12 for use in inhibiting colonisation of pathogenic gram positive bacteria on human skin or vagina.
